# EUROPEAN PATENT APPLICATION

(11) **EP 4 834 395 A1**
(43) Date of publication of application: **04.11.2026**
(21) Application number: 24910264.1
(22) Date of filing: 29.10.2024
(51) Int. Cl.: A61M 15/00

(54) **ROTATING MECHANISM, DOSAGE PROTECTION MECHANISM, BREATH TRIGGERING APPARATUS, AND DRY POWDER INHALER**

(30) Priority: 29.12.2023 CN 202311874076
(71) Applicant: Transpire Bio Inc, Sunrise, FL 33323 (US)
(72) Inventor: YI, Jie, Sunrise, FL 33323 (US)
(74) Representative: Latscha Schöllhorn Partner AG
(86) International application number: PCT/CN2024/128169
(87) International publication number: WO 2025/139325

(57) **Abstract**

A rotating mechanism (2), a dose protection mechanism (3), a breath triggering device (100), and a dry powder inhaler (800) are provided. The rotating mechanism (2) is used in the breath triggering device (100). The rotating mechanism (2) includes: a rotating shaft (22), where the outer side surface of the rotating shaft is provided with a notch (221); and a baffle (21), connected to the outer side surface of the rotating shaft (22). The rotating mechanism (2) is rotatable between a first position and a second position, and blocks a first airflow channel of the breath triggering device (100) when the rotating mechanism (2) is at the first position. When the rotating mechanism (2) moves from the first position to the second position, the baffle (21) rotates around the rotating shaft (22) to open the first airflow channel. Through the foregoing arrangement, the problem of a poor deaggregation effect on drug powder in the existing technology can be effectively resolved.

## Description

### CROSS-REFERENCE TO RELATED APPLICATIONS

This application claims priority to Chinese Patent Application No. 202311874076.2, filed on December 29, 2023, which is incorporated by reference in its entirety.

### TECHNICAL FIELD

This application relates to the technical field of inhalers, and in particular, to a rotating mechanism, a dose protection mechanism, a breath triggering device, and a dry powder inhaler.

### BACKGROUND

A blister-type dry powder inhaler usually draws, via airflow inhalation, drug powder from a dosage chamber to a mouthpiece, for a user to inhale. However, an existing blister-type dry powder inhalation device cannot guarantee protection on the drug powder in a case of low-speed inhalation via airflow, and a deaggregation effect of the airflow on the drug powder is poor during inhalation of a user, resulting in low utilization of the drug powder and easy waste of the drug powder.

### SUMMARY

This application mainly provides a rotating mechanism, a dose protection mechanism, a breath triggering device, and a dry powder inhaler, to resolve the problem in the existing technology that a deaggregation effect on drug powder in the dry powder inhaler is poor.

To resolve the foregoing technical problem, a technical solution used in this application is to provide a rotating mechanism, used in a breath triggering device. The rotating mechanism includes:
a rotating shaft, where the outer side surface of the rotating shaft is provided with a notch;
   and
a baffle, connected to the outer side surface of the rotating shaft.

The rotating mechanism is rotatable between a first position and a second position, and blocks a first airflow channel of the breath triggering device when the rotating mechanism is at the first position. When the rotating mechanism moves from the first position to the second position, the baffle rotates around the rotating shaft to open the first airflow channel.

A protruding rod is connected to the end of the rotating shaft.

The side surface of the end of the rotating shaft is provided with a groove, the end of the protruding rod is connected to the side wall of the groove, and an included angle is provided between the protruding rod and the baffle.

The baffle has the first surface, the first surface is provided with a flange, and the flange is arranged at the end of the baffle away from the rotating shaft, and extends from one side to the other side along the edge of the end of the baffle away from the rotating shaft.

A chamfer is provided on the end of the flange away from the rotating shaft.

The baffle further has the second surface opposite to the first surface, and the second surface is provided with a stop member. The stop member is arranged at the end of the baffle away from the rotating shaft, and extends from one side to the other side along the edge of the end of the baffle away from the rotating shaft. In the direction parallel to the second surface, the end of the stop member is connected to the second surface, and the other end of the stop member extends out of the second surface to form a limiting portion.

To resolve the foregoing technical problem, a technical solution used in this application is to provide a dose protection mechanism, used in a breath triggering device.

The dose protection mechanism is movable between a third position and a fourth position. The dose protection mechanism includes a plate-shaped portion, and the plate-shaped portion is provided with a first communication hole and a second communication hole spaced apart from each other.

When the dose protection mechanism is at the third position, the plate-shaped portion blocks a second airflow channel of the breath triggering device, and the second communication hole is in communication with external air and an airway chamber of the breath triggering device. When the dose protection mechanism is at the fourth position, the first communication hole is in communication with the second airflow channel, and the plate-shaped portion is used for blocking the airway chamber.

The dose protection mechanism further includes a body portion, and a limiting member is provided on the body portion.

The limiting member is a protruding post obliquely arranged on the surface of the body portion, and the protruding post has the free end.

The first communication hole and the second communication hole are circular holes, and are aligned with each other and spaced apart in the movement direction of the dose protection mechanism.

The body portion is further provided with an accommodation groove.

To resolve the foregoing technical problem, another technical solution used in this application is to provide a breath triggering device, including:
an airway chamber;
a rotating mechanism, rotatable between a first position and a second position; and
a dose protection mechanism, movable between a third position and a fourth position.

When a negative pressure in the airway chamber of the breath triggering device is greater than a preset threshold, external air pressure pushes the rotating mechanism to rotate from the first position to the second position, the rotating mechanism releases a limit on the dose protection mechanism, and the dose protection mechanism moves from the third position to the fourth position; and/or
when the negative pressure in the airway chamber of the breath triggering device is greater than the preset threshold, the rotating mechanism and the dose protection mechanism move, for a communication manner of an airflow channel of the breath triggering device to be changed.

The rotating mechanism is the foregoing rotating mechanism.

The dose protection mechanism is the foregoing dose protection mechanism.

The breath triggering device further includes a dosage chamber spaced apart from the airway chamber, where the airway chamber is in communication with the dosage chamber via the second airflow channel, and the airway chamber is in communication with the external air via the first airflow channel or a third airflow channel.

When a negative pressure in the airway chamber is greater than the preset threshold, the external air pressure pushes the rotating mechanism to rotate from the first position to the second position, for the first airflow channel to be opened. When the dose protection mechanism is at the third position, the second airflow channel is blocked, the airway chamber is not in communication with the dosage chamber, the third airflow channel is opened, and the airway chamber is in communication with the external air via the third airflow channel. When the dose protection mechanism is at the fourth position, the second airflow channel is opened, to communicate the airway chamber with the dosage chamber, and the third airflow channel is blocked.

The breath triggering device further includes:
a support, provided with the airway chamber and the dosage chamber spaced apart from each other, where the rotating mechanism is rotatably arranged on the support, and the dose protection mechanism is movably arranged on the support; and
an elastic member, arranged in contact with the dose protection mechanism, and configured to drive the dose protection mechanism to move from the third position to the fourth position.

When the rotating mechanism rotates from the first position to the second position, the rotating mechanism releases the limit on the dose protection mechanism, for the elastic member to drive the dose protection mechanism to move from the third position to the fourth position.

The dosage chamber is provided with a first powder discharge hole, and the airway chamber is provided with a second powder discharge hole. The first powder discharge hole and the second powder discharge hole are aligned with each other and spaced apart, to form the part of the second airflow channel. The support is further provided with a third communication hole, and the third communication hole and the airway chamber are spaced apart from each other.

When the dose protection mechanism is at the third position, the first communication hole is staggered with the first powder discharge hole, and the first powder discharge hole is blocked. When the dose protection mechanism is at the third position, the second communication hole is in alignment and communication with the third communication hole to form the part of the third airflow channel. When the dose protection mechanism is at the fourth position, the first communication hole is located between the first powder discharge hole and the second powder discharge hole, for the first powder discharge hole, the first communication hole, and the second powder discharge hole to be in alignment and communication sequentially to form the second airflow channel.

The breath triggering device further includes a translation mechanism. The translation mechanism is movably arranged on the support and is movable between a fifth position and a sixth position. The translation mechanism is separately arranged in contact with the rotating mechanism and the dose protection mechanism.

The translation mechanism moves from the sixth position to the fifth position, to drive the dose protection mechanism to be reset from the fourth position to the third position, and drive the rotating mechanism to be reset from the second position to the first position.

The dose protection mechanism is slidably arranged on the support. The dose protection mechanism includes the body portion, and the plate-shaped portion is connected to the side of the body portion away from the elastic member. The end of the rotating shaft is connected to the protruding rod, and the body portion is provided with the accommodation groove.

The translation mechanism includes a support member and a push rod connected to each other, the support member abuts against the protruding rod, and the push rod is at least partially arranged in the accommodation groove.

When the translation mechanism moves from the sixth position to the fifth position, the support member pushes the protruding rod, to reset the rotating mechanism from the second position to the first position, and the push rod pushes the body portion, to reset the dose protection mechanism from the fourth position to the third position.

The airway chamber and the dosage chamber are spaced apart from each other in the first direction. The first airflow channel and the rotating mechanism are arranged on the side of the airway chamber in the second direction. The dose protection mechanism slides in the second direction. The shaft direction of the rotating shaft is the third direction.

The first direction, the second direction, and the third direction are perpendicular to each other.

To resolve the foregoing technical problem, another technical solution used in this application is to provide a dry powder inhaler, including:
a housing assembly, including a housing and a mouthpiece; and
a breath triggering device, arranged in the housing, where the breath triggering device is the breath triggering device of any one of the foregoing descriptions.

The mouthpiece is in communication with the airway chamber.

In some implementations, the dry powder inhaler further includes:
a cover body, rotatably connected to the housing assembly, where the cover body is rotatable between a seventh position and an eighth position, and drives the translation mechanism to move from the sixth position to the fifth position when the cover body rotates from the eighth position to the seventh position; when the cover body is at the seventh position, the cover body covers the mouthpiece and limits the rotating mechanism to the first position via the translation mechanism; and when the cover body is at the eighth position, the cover body exposes the mouthpiece and releases the limit on the translation mechanism.

In some implementations, the cover body is connected to the translation mechanism, and drives the translation mechanism to slide between the fifth position and the sixth position based on that the cover body rotates between the seventh position and the eighth position; or
the support is further provided with an elastic arm, where the elastic arm abuts against the translation mechanism when the translation mechanism is at the fifth position; and the elastic arm drives the translation mechanism to slide from the fifth position to the sixth position when the cover body is at the eighth position; or
the translation mechanism is driven to slide from the fifth position to the sixth position when the rotating mechanism rotates from the first position to the second position; or
the translation mechanism is driven to slide from the fifth position to the sixth position when the dose protection mechanism moves from the third position to the fourth position.

Beneficial effects of this application are as follows: Different from the case in the existing technology, this application discloses a rotating mechanism, a dose protection mechanism, a breath triggering device, and a dry powder inhaler. The rotating mechanism is used in the breath triggering device. The rotating mechanism includes: a rotating shaft, where the outer side surface of the rotating shaft is provided with a notch; and a baffle, connected to the outer side surface of the rotating shaft. The rotating mechanism is rotatable between a first position and a second position, and blocks a first airflow channel of the breath triggering device when the rotating mechanism is at the first position. When the rotating mechanism moves from the first position to the second position, the baffle rotates around the rotating shaft to open the first airflow channel. Through the foregoing arrangement, the problem of a poor deaggregation effect on drug powder in the existing technology can be effectively resolved.

### BRIEF DESCRIPTION OF THE DRAWINGS

To describe the technical solutions of the embodiments of this application or the existing technology more clearly, the following briefly introduces the accompanying drawings required for describing the embodiments or the existing technology. Apparently, the accompanying drawings in the following description show some embodiments recorded in this application, and a person of ordinary skill in the art still derives other drawings from these accompanying drawings without creative efforts.
FIG. 1 is a schematic structural diagram of a dry powder inhaler in a first state according to this application;
FIG. 2 is a schematic exploded view of the dry powder inhaler provided in FIG. 1;
FIG. 3 is a schematic structural diagram of a breath triggering device when the dry powder inhaler provided in FIG. 1 is in the first state;
FIG. 4A is a cross sectional schematic view of the breath triggering device provided in FIG. 3;
FIG. 4B is a partially enlarged schematic view of FIG. 4A;
FIG. 5 is another cross sectional schematic view of the breath triggering device provided in FIG. 3;
FIG. 6 is a schematic structural diagram of a dry powder inhaler in a second state according to this application;
FIG. 7 is a schematic structural diagram of a breath triggering device when the dry powder inhaler provided in FIG. 6 is in the second state;
FIG. 8A is a cross sectional schematic view of the breath triggering device provided in FIG. 7;
FIG. 8B is a partially enlarged schematic view of FIG. 8A;
FIG. 9A is another cross sectional schematic view of the breath triggering device provided in FIG. 7;
FIG. 9B is a partially enlarged schematic view of FIG. 9A;
FIG. 10 is a schematic structural diagram of a mouthpiece of the dry powder inhaler provided in FIG. 1;
FIG. 11 is a schematic structural diagram of a support of the breath triggering device provided in FIG. 3 from a perspective;
FIG. 12 is a schematic structural diagram of the support provided in FIG. 11 from another perspective;
FIG. 13 is a partially enlarged schematic view of a cross section of the support provided in FIG. 11;
FIG. 14 is a partially enlarged schematic view of another cross section of the support provided in FIG. 11;
FIG. 15 is a schematic structural diagram of a rotating mechanism of the breath triggering device provided in FIG. 3 from a perspective;
FIG. 16 is a schematic structural diagram of the rotating mechanism provided in FIG. 15 from another perspective;
FIG. 17 is a schematic structural diagram of a dose protection mechanism of the breath triggering device provided in FIG. 3;
FIG. 18 is a schematic structural diagram of a translation mechanism of the breath triggering device provided in FIG. 3;
FIG. 19 is a schematic structural diagram of an embodiment of an airway structure of a dry powder inhaler according to this application;
FIG. 20 is a cross sectional schematic view of the airway structure provided in FIG. 19;
FIG. 21 is another cross sectional schematic view of the airway structure provided in FIG. 19;
FIG. 22 is a schematic structural diagram of an airway chamber of the airway structure provided in FIG. 19;
FIG. 23 is a top schematic structural diagram of a manifold of the airway structure provided in FIG. 19;
FIG. 24 is a bottom schematic structural diagram of the manifold provided in FIG. 23;
FIG. 25 is a schematic structural diagram of a cover body of the dry powder inhaler provided in FIG. 1;
FIG. 26 is a schematic structural diagram of an embodiment of a take-up device according to this application;
FIG. 27 is a cross sectional schematic view of the take-up device provided in FIG. 25;
FIG. 28 is a schematic structural diagram of a tape take-up rod of the take-up device provided in FIG. 25;
FIG. 29 is a bottom schematic structural diagram of a tape take-up gear of the take-up device provided in FIG. 25;
FIG. 30 is a bottom schematic structural diagram of the tape take-up gear provided in FIG. 29; and
FIG. 31 is a schematic structural diagram of a wave washer of the take-up device provided in FIG. 25.

### Reference numerals:

dry powder inhaler 800; breath triggering device 100; support 1; third air inlet 10; airway chamber 11; second powder discharge hole 111; annular side wall 112; first side wall 1121; second side wall 1122; third side wall 1123; fourth side wall 1124; bottom wall 113; top wall 114; first fin 115; second fin 116; first airway portion 117; second airway portion 118; mixed airway portion 119; vortex airway 110; dosage chamber 12; first powder discharge hole 121; vent hole 122; first airflow channel Q1; second airflow channel Q2; third airflow channel Q3; air inlet channel Q4; first direction A1; second direction A2; third direction A3; elastic arm 13; third communication hole 14; air outlet hole 15; first air inlet 16; second air inlet 17; support body 18; manifold 19; insertion portion 191; annular insertion segment 1911; first insertion segment 1912; second insertion segment 1913; covering portion 192; communicating portion 193; rotating mechanism 2; baffle 21; first surface 211; flange 213; second surface 212; stop member 214; limiting portion 2141; rotating shaft 22; notch 221; groove 222; protruding rod 23; dose protection mechanism 3; plate-shaped portion 31; first communication hole 311; second communication hole 312; limiting member 32; body portion 33; accommodation groove 331; cutout 34; elastic member 4; translation mechanism 5; support member 51; support portion 511; connection portion 512; push rod 52; projecting portion 53; drug tape 6; base tape 61; medicament accommodating portion 62; drug tape accommodating groove 7; housing assembly 200; housing 201; external air inlet 2011; grille 2012; first housing 206; second housing 207; first protruding portion 208; second protruding portion 209; mouthpiece 202; annular surrounding portion 203; surrounding wall 204; depressed portion 205; cover body 300; arc-shaped portion 301; rotating portion 302; supply reel 400; drug tape mounting shaft 401; cover take-up reel 500; dosage wheel 600; base take-up reel 700; take-up device 900; tape take-up rod 91; rod-shaped portion 911; first clamping portion 912; second clamping portion 913; first rod segment 914; second rod segment 915; first slot 916; second slot 917; tape take-up gear 92; central hole 921; limiting groove 922; receiving groove 923; washer 93; rigid washer 931; and elastic washer 932.

### DETAILED DESCRIPTION

The following clearly and completely describes the technical solutions in the embodiments of this application with reference to the drawings in the embodiments of this application. Apparently, the described embodiments are some embodiments of this application rather than all of the embodiments. All other embodiments obtained by a person skilled in the art based on the embodiments of this application without creative efforts shall fall within the protection scope of this application.

In the embodiments of this application, terms "first", "second", and "third" are used merely for the purpose of description, and shall not be construed as indicating or implying relative importance or implying a quantity of indicated technical features. Therefore, features defining "first", "second", and "third" can explicitly or implicitly include at least one of the features. In the descriptions of this application, unless explicitly specified, "a plurality of" means at least two, for example, two or three. In addition, the terms "include", "have", and any variant thereof are intended to cover a non-exclusive inclusion. For example, a process, method, system, product, or device that includes a series of steps or units is not limited to the listed steps or units; and instead, further optionally includes a step or unit that is not listed, or further optionally includes another step or unit that is intrinsic to the process, method, product, or device.

"Embodiment" mentioned in this specification means that particular features, structures, or characteristics described with reference to the embodiment may be included in at least one embodiment of this application. The term appearing at different positions of this specification may not refer to the same embodiment or an independent or alternative embodiment that is mutually exclusive with another embodiment. It shall be explicitly and implicitly understood by a person skilled in the art that the embodiments described herein may be combined with other embodiments.

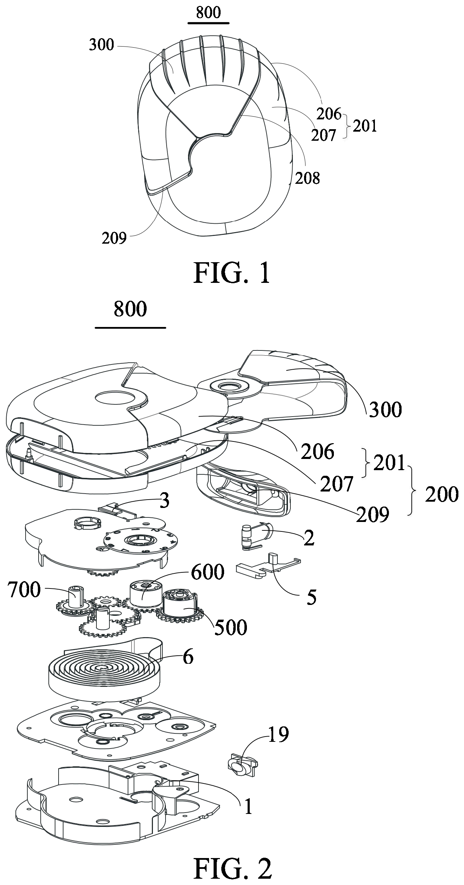

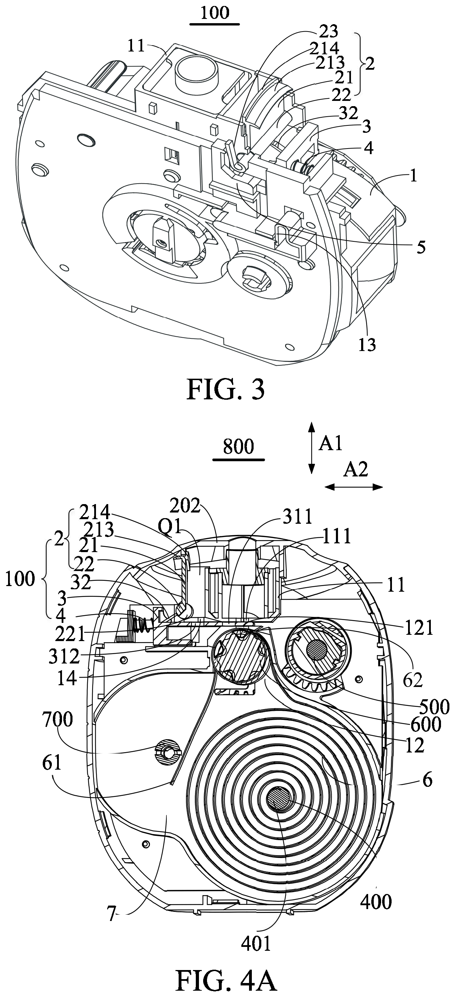

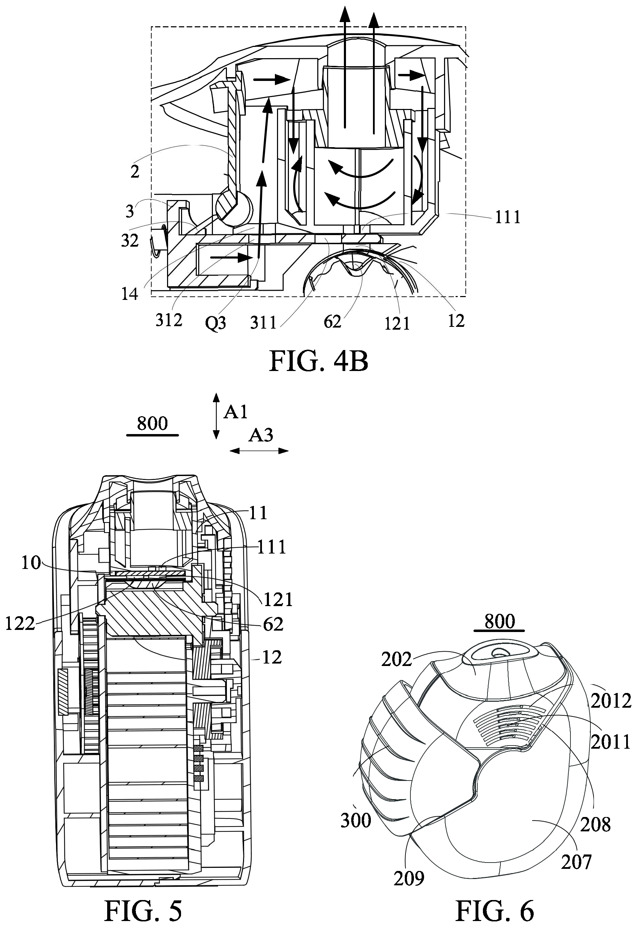

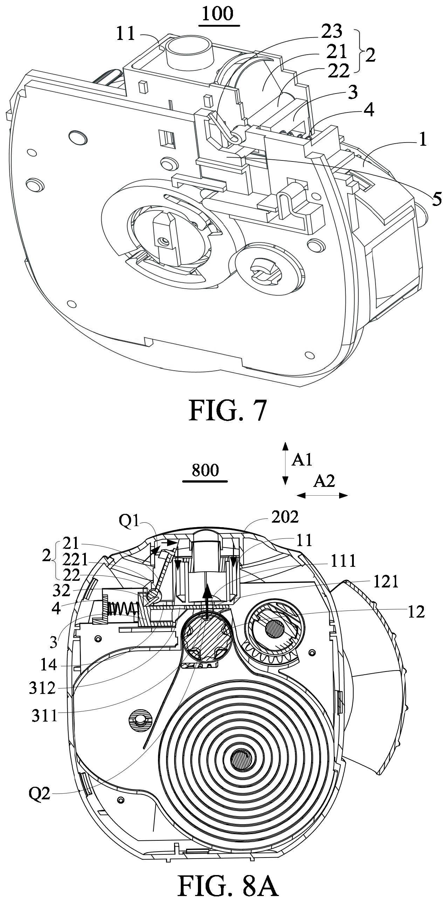

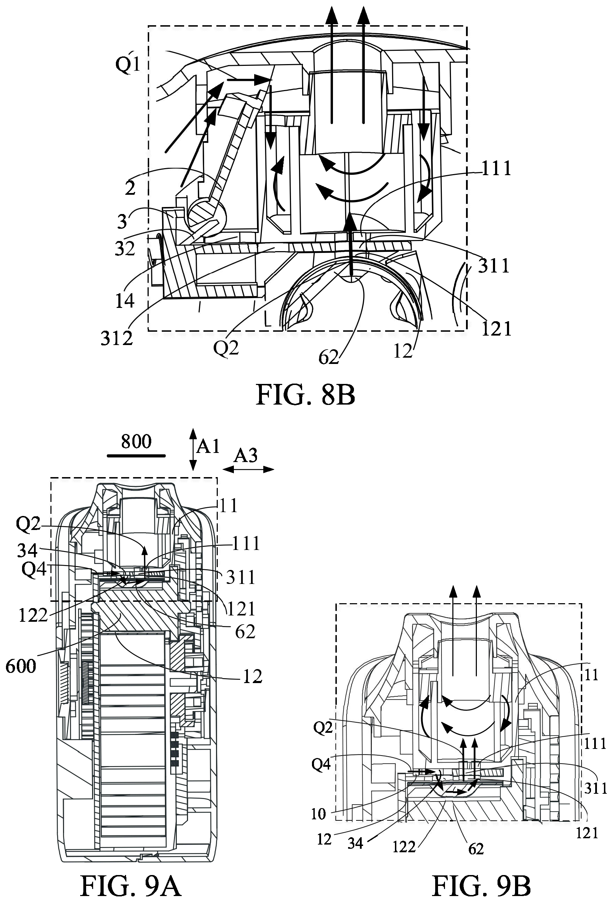

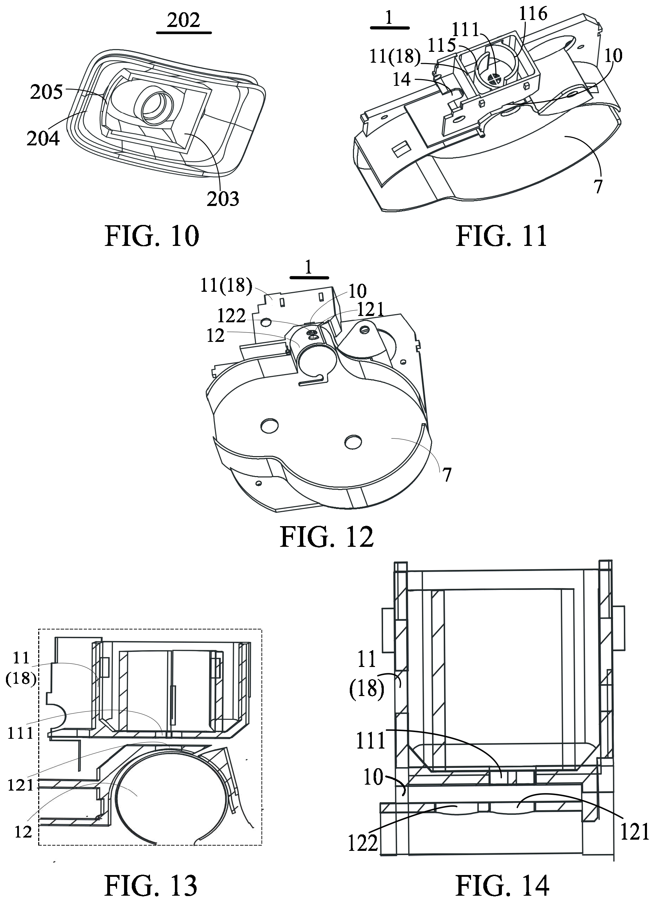

Referring to FIG. 1 to FIG. 10, FIG. 1 is a schematic structural diagram of a dry powder inhaler in a first state according to this application; FIG. 2 is a schematic exploded view of the dry powder inhaler provided in FIG. 1; FIG. 3 is a schematic structural diagram of a breath triggering device when the dry powder inhaler provided in FIG. 1 is in the first state; FIG. 4A is a cross sectional schematic view of the breath triggering device provided in FIG. 3; FIG. 4B is a partially enlarged schematic view of FIG. 4A; FIG. 5 is another cross sectional schematic view of the breath triggering device provided in FIG. 3; FIG. 6 is a schematic structural diagram of a dry powder inhaler in a second state according to this application; FIG. 7 is a schematic structural diagram of a breath triggering device when the dry powder inhaler provided in FIG. 6 is in the second state; FIG. 8A is a cross sectional schematic view of the breath triggering device provided in FIG. 7; FIG. 8B is a partially enlarged schematic view of FIG. 8A; FIG. 9A is another cross sectional schematic view of the breath triggering device provided in FIG. 7; FIG. 9B is a partially enlarged schematic view of FIG. 9A; and FIG. 10 is a schematic structural diagram of a mouthpiece of the dry powder inhaler provided in FIG. 1.

Referring to FIG. 1 and FIG. 2, this application provides a dry powder inhaler 800. The dry powder inhaler 800 includes a housing assembly 200, a breath triggering device 100, and a cover body 300. The cover body 300 is rotatably connected to the housing assembly 200. The housing assembly 200 includes a housing 201 and a mouthpiece 202. The housing 201 is connected to the mouthpiece 202. The breath triggering device 100 is arranged in the housing 201. The dry powder inhaler 800 may perform conversion between a first state and a second state. When the dry powder inhaler 800 is separately in the first state and the second state, the cover body 300 is located at different positions. Specifically, when the dry powder inhaler 800 is in the first state, the cover body 300 is in a closed state, and the cover body 300 covers the mouthpiece 202. When the dry powder inhaler 800 is in the second state, the cover body 300 is in an open state, and the cover body 300 exposes the mouthpiece 202, so that a user can inhale drug powder at the mouthpiece 202. The first state of the dry powder inhaler 800 is a cover-closed state, and the second state is a state in which a cover is completely opened.

Referring to FIG. 1, FIG. 2, FIG. 3, FIG. 6, and FIG. 7, in a specific implementation, the housing 201 includes a first housing 206 and a second housing 207 connected to each other. The first housing 206 and the second housing 207 cooperate to form an accommodating space for accommodating a structural member like the breath triggering device 100. The mouthpiece 202 is connected to the end of the housing 201. Referring to FIG. 6, the housing 201 is provided with an external air inlet 2011, and the external air inlet 2011 may be provided on the first housing 206 and/or the second housing 207. As shown in FIG. 1, when the dry powder inhaler 800 is in the first state, that is, the cover-closed state, the cover body 300 is closed, and the cover body 300 covers the external air inlet 2011 and the mouthpiece 202. As shown in FIG. 6, when the dry powder inhaler 800 is in the second state, that is, the open state, the cover body 300 is opened, the external air inlet 2011 and the mouthpiece 202 are exposed, and external air may enter the housing assembly 200 via the external air inlet 2011, and specifically, enter the accommodating space formed by cooperation between the first housing 206 and the second housing 207. Further, referring to FIG. 6, the housing 201 may be further provided with a grille 2012, and the grille 2012 is projected out of the outer surface of the housing 201, to prevent the problem that air intake is poor because the lips block the external air inlet 2011 when the user inhales at the mouthpiece 202. In a specific implementation, the housing 201 may be provided with a plurality of external air inlets 2011 and a plurality of grilles 2012, and the external air inlets 2011 and the grilles 2012 may be alternately arranged, to better prevent the external air inlets 2011 from being blocked by the lips, thereby preventing poor air intake.

Specifically, referring to FIG. 3 to FIG. 9B, the breath triggering device 100 includes a support 1, a rotating mechanism 2, a dose protection mechanism 3, and an elastic member 4. The support 1 is provided with an airway chamber 11 and a dosage chamber 12 spaced apart from each other. The airway chamber 11 is in communication with the external air via a first airflow channel Q1. The airway chamber 11 is in communication with the dosage chamber 12 via a second airflow channel Q2. The mouthpiece 202 is in communication with the airway chamber 11. The rotating mechanism 2 is rotatably arranged on the support 1, and is rotatable between a first position and a second position. When the dry powder inhaler 800 is in the first state, that is, the cover-closed state, the cover body 300 is in the closed state, the rotating mechanism 2 is at the first position, the rotating mechanism 2 blocks the port of the first airflow channel Q1, and the airway chamber 11 cannot be in communication with the external air via the first airflow channel Q1. When the dry powder inhaler 800 is in the second state, that is, the state in which the cover is completely opened, and a negative pressure in the airway chamber 11 is greater than a preset threshold, that is, a flow rate of airflow during inhalation of the user is greater than a working threshold, external air pressure pushes the rotating mechanism 2 to rotate from the first position to the second position, so that the port of the first airflow channel Q1 is opened. The first position is an initial position of the rotating mechanism 2 when the breath triggering device 100 is not triggered. The second position is a final position of the rotating mechanism 2 after the breath triggering device 100 is triggered. To be specific, when the flow rate of the airflow during inhalation of the user is sufficiently large, so that the negative pressure in the airway chamber 11 is greater than the preset threshold, under a pressure difference between the external air pressure and air pressure in the airway chamber 11, the external air pressure pushes the rotating mechanism 2 to rotate, so that the port of the first airflow channel Q1 is opened, and the first airflow channel Q1 is in communication with the external air via the port. However, when the flow rate of the airflow inhaled by the user is relatively small, and the negative pressure in the airway chamber 11 is less than or equal to the preset threshold, the rotating mechanism 2 does not rotate under the pushing action of the external air pressure, so that the port of the first airflow channel Q1 is not opened.

The dose protection mechanism 3 is movably arranged on the support 1, and is movable between a third position and a fourth position. When the dose protection mechanism 3 is at the third position, the dose protection mechanism 3 blocks the second airflow channel Q2. When the dose protection mechanism 3 is at the fourth position, the second airflow channel Q2 is opened, so that the airway chamber 11 is in communication with the dosage chamber 12. Specifically, the airway chamber 11 is in communication with a medicament accommodating portion 62 of a base tape 61 in the dosage chamber 12. The third position is an initial position of the dose protection mechanism 3 when the breath triggering device 100 is not triggered. The fourth position is a final position of the dose protection mechanism 3 after the breath triggering device 100 is triggered. The elastic member 4 is arranged in contact with the dose protection mechanism 3, and configured to drive the dose protection mechanism 3 to move from the third position to the fourth position. When the rotating mechanism 2 is at the first position, the rotating mechanism 2 limits the dose protection mechanism 3 to the third position. When the rotating mechanism 2 rotates from the first position to the second position, the rotating mechanism 2 releases the limit on the dose protection mechanism 3, so that the elastic member 4 can drive the dose protection mechanism 3 to move from the third position to the fourth position. The elastic member 4 may be a spring or an elastic component in any other structure.

It may be understood that, the breath triggering device 100 is arranged in the dry powder inhaler 800, the airway chamber 11 in the breath triggering device 100 is in communication with the medicament accommodating portion 62 in the dosage chamber 12 via the second airflow channel Q2, the airway chamber 11 is in communication with the external air via the first airflow channel Q1, and the rotating mechanism 2 is rotatably arranged on the support 1. In this way, only when the flow rate of the airflow during inhalation of the user is sufficiently large and the negative pressure in the airway chamber 11 is greater than the preset threshold, the rotating mechanism 2 rotates from the first position to the second position under the pushing action of the external air pressure, so that the port of the first airflow channel Q1 is opened, and the rotating mechanism 2 releases the limit on the dose protection mechanism 3. In this way, the dose protection mechanism 3 can move from the third position to the fourth position under the action of the elastic member 4, so that the second airflow channel Q2 is opened, and the airway chamber 11 can be in communication with the dosage chamber 12 via the second airflow channel Q2, so that drug powder in the medicament accommodating portion 62 in the dosage chamber 12 can enter the airway chamber 11 via the second airflow channel Q2, can be deaggregated by air, and then can be inhaled by the user at the mouthpiece 202. In other words, the breath triggering device 100 is arranged, so that the second airflow channel Q2 is opened only when the flow rate of the airflow during inhalation of the user is sufficiently large, thereby ensuring, during inhalation of the user, a deaggregation effect of high-flow-rate airflow on the drug powder in the medicament accommodating portion 62 in the dosage chamber 12, improving utilization of the drug powder, reducing waste of the drug powder, and resolving the problem of a poor deaggregation effect on drug powder in a dry powder inhaler in the existing technology.

In an embodiment, the breath triggering device 100 further includes a translation mechanism 5. The translation mechanism 5 is movably arranged on the support 1 and is movable between a fifth position and a sixth position. The translation mechanism 5 is separately arranged in contact with the rotating mechanism 2 and the dose protection mechanism 3. When the translation mechanism 5 moves from the sixth position to the fifth position, the translation mechanism 5 can drive the dose protection mechanism 3 to be reset from the fourth position to the third position, and drive the rotating mechanism 2 to be reset from the second position to the first position. In other words, the translation mechanism 5 may move to drive the rotating mechanism 2 and the dose protection mechanism 3 to be reset.

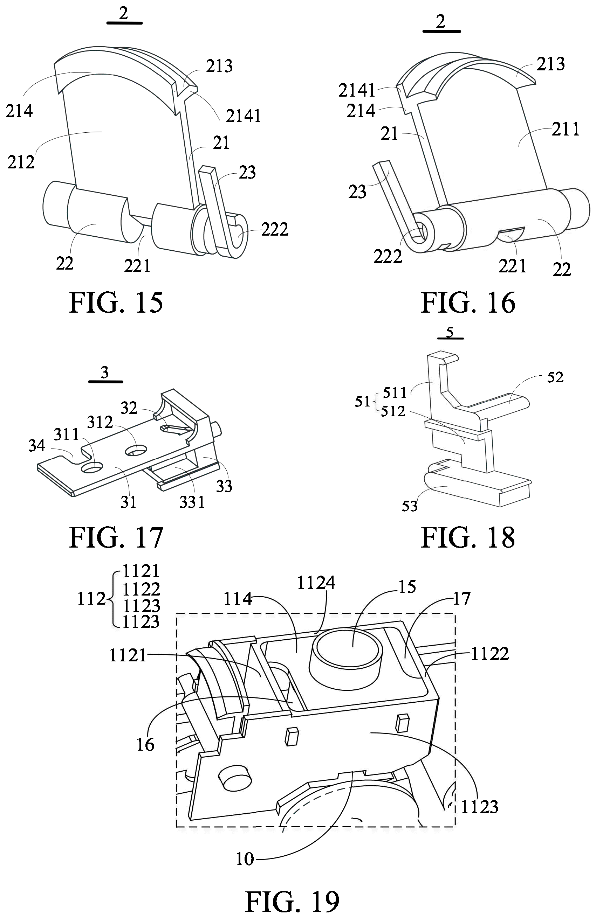

Referring to FIG. 11 to FIG. 18, FIG. 11 is a schematic structural diagram of a support of the breath triggering device provided in FIG. 3 from a perspective; FIG. 12 is a schematic structural diagram of the support provided in FIG. 11 from another perspective; FIG. 13 is a partially enlarged schematic view of a cross section of the support provided in FIG. 11; FIG. 14 is a partially enlarged schematic view of another cross section of the support provided in FIG. 11; FIG. 15 is a schematic structural diagram of a rotating mechanism of the breath triggering device provided in FIG. 3 from a perspective; FIG. 16 is a schematic structural diagram of the rotating mechanism provided in FIG. 15 from another perspective; FIG. 17 is a schematic structural diagram of a dose protection mechanism of the breath triggering device provided in FIG. 3; and FIG. 18 is a schematic structural diagram of a translation mechanism of the breath triggering device provided in FIG. 3.

Referring to FIG. 4A, FIG. 4B, FIG. 8A, and FIG. 8B, the airway chamber 11 is provided with a second powder discharge hole 111, and the dosage chamber 12 is provided with a first powder discharge hole 121. The first powder discharge hole 121 and the second powder discharge hole 111 are aligned with each other and spaced apart, to form the part of the second airflow channel Q2. It should be noted that, the "aligned" in this application may be completely aligned, or may be partially aligned. When projections in a direction at least partially overlap with each other, this may be referred to as alignment. For example, the first powder discharge hole 121 and the second powder discharge hole 111 are aligned with each other, the diameter of the first powder discharge hole 121 may be equal to the diameter of the second powder discharge hole 111, and projections of the first powder discharge hole 121 and the second powder discharge hole 111 completely overlap with each other, that is, the first powder discharge hole 121 and the second powder discharge hole 111 are completely aligned with each other. Alternatively, the diameter of the first powder discharge hole 121 is equal to the diameter of the second powder discharge hole 111, and the projections of the first powder discharge hole 121 and the second powder discharge hole 111 only partially overlap with each other, so that the parts of the first powder discharge hole 121 and the second powder discharge hole 111 are aligned with each other, and the other parts of the first powder discharge hole 121 and the second powder discharge hole 111 are staggered with each other. The diameter of the first powder discharge hole 121 is greater than the diameter of the second powder discharge hole 111, the projection of the second powder discharge hole 111 falls within a range of the projection of the first powder discharge hole 121, and the projections of the first powder discharge hole 121 and the second powder discharge hole 111 partially overlap with each other, so that the first powder discharge hole 121 and the second powder discharge hole 111 are partially aligned with each other. Alternatively, the diameter of the first powder discharge hole 121 is greater than the diameter of the second powder discharge hole 111, and the projection of the second powder discharge hole 111 only partially falls within the range of the projection of the first powder discharge hole 121, so that the first powder discharge hole 121 and the second powder discharge hole 111 are partially aligned with each other. The plurality of cases may be referred to as that the first powder discharge hole 121 and the second powder discharge hole 111 are aligned with each other. Preferably, the shapes of the first powder discharge hole 121 and the second powder discharge hole 111 are the same. For example, if the first powder discharge hole 121 and the second powder discharge hole 111 are circular holes, the first powder discharge hole 121 and the second powder discharge hole 111 have the same diameter and are completely aligned with each other. This is more beneficial for the drug powder to enter the second powder discharge hole 111 from the first powder discharge hole 121, thereby reducing waste of the drug powder.

Specifically, the top wall 114 of the dosage chamber 12 and the bottom wall 113 of the airway chamber 11 are spaced apart from each other, the second powder discharge hole 111 is provided on the bottom wall 113 of the airway chamber 11, and the first powder discharge hole 121 is provided on the top wall 114 of the dosage chamber 12. Referring to FIG. 17, the dose protection mechanism 3 includes a plate-shaped portion 31, and the plate-shaped portion 31 is at least partially movably arranged between the top wall 114 of the dosage chamber 12 and the bottom wall 113 of the airway chamber 11. The plate-shaped portion 31 is provided with a first communication hole 311. When the dose protection mechanism 3 is at the third position, the first communication hole 311 is staggered with the first powder discharge hole 121, and the dose protection mechanism 3 blocks the first powder discharge hole 121. Specifically, the plate-shaped portion 31 blocks the first powder discharge hole 121, so that the second airflow channel Q2 cannot be in communication. When the dose protection mechanism 3 is at the fourth position, the first communication hole 311 is located between the first powder discharge hole 121 and the second powder discharge hole 111, so that the first powder discharge hole 121, the first communication hole 311, and the second powder discharge hole 111 are in alignment and communication sequentially to form the second airflow channel Q2. In other words, when the dose protection mechanism 3 is at the third position, the second airflow channel Q2 cannot be in communication, and when the dose protection mechanism 3 is at the fourth position, the second airflow channel Q2 is in communication. A motion state of the rotating mechanism 2 is determined based on a relationship between the negative pressure in the airway chamber 11 and the preset threshold, to control a moving state of the dose protection mechanism 3 between the third position and the fourth position, thereby implementing switching between a blocked state and a communicated state of the second airflow channel Q2. In this way, only when the flow rate of the airflow during inhalation of the user is sufficiently large and the negative pressure in the airway chamber 11 is greater than the preset threshold, the second airflow channel Q2 is in communication, and the drug powder in the first powder discharge hole 121 is inhaled. This is more beneficial to deaggregation of the airflow on the drug powder, thereby improving the utilization of the drug powder. In addition, when the dose protection mechanism 3 is at the third position, the drug powder is still processed in a relatively closed state, thereby preventing the drug powder from leaking out or being polluted.

Referring to FIG. 3, FIG. 4A, FIG. 4B, FIG. 7, FIG. 8A, FIG. 8B, and FIG. 15 to FIG. 17, the rotating mechanism 2 includes a baffle 21 and a rotating shaft 22 connected to each other. The rotating shaft 22 is rotatably connected to the support 1. The baffle 21 is configured to block the first airflow channel Q1. In a specific implementation, as shown in FIG. 3, FIG. 7, FIG. 15, and FIG. 16, the baffle 21 has a flat plate shape. The baffle 21 has the first surface 211 and the second surface 212 opposite to each other. The first surface 211 is the surface of the baffle 21 far away from the airway chamber 11, and the second surface 212 is the surface of the baffle 21 close to the airway chamber 11. The first surface 211 is provided with a flange 213, the second surface 212 is provided with a stop member 214, and the shape of the flange 213 is consistent with the cross sectional shape of the side wall of the port of the first airflow channel Q1, so that the flange 213 is better engaged with the side wall of the port of the first airflow channel Q1 to block the port of the first airflow channel Q1. The flange 213 is located on the side of the baffle 21 away from the airway chamber 11, the stop member 214 is located on the side of the baffle 21 close to the airway chamber 11, and the flange 213 and the stop member 214 are both arranged at the end of the baffle 21 away from the rotating shaft 22. The end of the stop member 214 is connected to the second surface 212, and the other end of the stop member 214 extends out of the second surface 212 in the direction parallel to the second surface 212 and forms a limiting portion 2141. Specifically, the size of the flange 213 is smaller than the size of the stop member 214 in the direction parallel to the second surface 212, the size of the flange 213 is larger than the size of the stop member 214 in the direction perpendicular to the baffle 21, and the flange 213 and the stop member 214 extend from the side of the baffle 21 to the other side of the baffle 21 in the shaft direction of the rotating shaft 22. When the rotating mechanism 2 is at the first position, the flange 213 and the stop member 214 may be configured to cooperate with the mouthpiece 202 to block the first airflow channel Q1.

Specifically, as shown in FIG. 3, FIG. 4A, FIG. 4B, FIG. 8A, FIG. 8B, and FIG. 10, an annular surrounding portion 203 is connected to the surface of the mouthpiece 202 close to the airway chamber 11. The annular surrounding portion 203 is configured to cooperate with the airway chamber 11 and the support 1 to form the first airflow channel Q1. The annular surrounding portion 203 includes a surrounding wall 204. The surrounding wall 204 and the airway chamber 11 are spaced apart from each other. The support 1, the rotating mechanism 2, the annular surrounding portion 203, and the airway chamber 11 jointly enclose the first airflow channel Q1. The surrounding wall 204 is the side wall of the port of the first airflow channel Q1. The rotating mechanism 2 is arranged at the port of the first airflow channel Q1. A depressed portion 205 is provided on the end surface of the surrounding wall 204 close to the dose protection mechanism 3. In a specific implementation, the depressed portion 205 is arc-shaped, and the flange 213 is arc-shaped. When the rotating mechanism 2 is at the first position, that is, an initial position of the rotating mechanism 2 when the breath triggering device 100 is not triggered, the baffle 21 and the surrounding wall 204 are completely aligned with each other, and the surface of the flange 213 away from the rotating shaft 22 is an arc surface, and abuts against the bottom surface of the depressed portion 205 to block the port of the first airflow channel Q1. In a process in which the baffle 21 rotates from the first position to the second position, the port of the first airflow channel Q1 is opened, and after the external air enters the inside of the housing 201, the external air enters the first airflow channel Q1 via a gap between the flange 213 of the baffle 21 and the bottom of the depressed portion 205 of the surrounding wall 204, and further enters the inside of the airway chamber 11 via the first airflow channel Q1, facilitating better deaggregation on the drug powder in the airway chamber 11.

It may be understood that, the flange 213 projects out of the first surface 211, and in a process in which the baffle 21 rotates around the rotating shaft 22 from the first position to the second position, the flange 213 can ensure that cross-sectional areas for intake when the external air enters the first airflow channel Q1 are basically consistent in the entire process, that is, distances between the flange 213 and the bottom wall of the depressed portion 205 are basically consistent, thereby ensuring consistency of inhalation resistance during entire movement of the rotating mechanism 2, and avoiding a sudden change in inhalation resistance of the dry powder inhaler 800 when the baffle 21 rotates because the flange 213 is not arranged. Only when the flow rate of the airflow during inhalation of the user is sufficiently large and the negative pressure in the airway chamber 11 is sufficiently large, the baffle 21 rotates around the rotating shaft 22 to rotate by a larger angle, and the flange 213 completely rotates to be out of contact with the bottom surface of the depressed portion 205 of the surrounding wall 204, so that the port of the first airflow channel Q1 is opened, and the first airflow channel Q1 is in communication with the external air. In other words, the flange 213 can further ensure that the first airflow channel Q1 is opened when the flow rate of the airflow during inhalation of the user is sufficiently large and the negative pressure in the airway chamber 11 is greater than the preset threshold, thereby improving the deaggregation effect on the drug powder, and avoiding waste of the drug powder. In addition, the flange 213 arranged on the first surface 211 can also have a particular air concentration effect.

Further, in some implementations, as shown in FIG. 15 and FIG. 16, a chamfer is further provided on the end of the flange 213 away from the rotating shaft 22. The chamfer may be an oblique angle, or may be a rounded angle. It may be understood that, providing the chamfer on the end of the flange 213 away from the rotating shaft 22 can effectively avoid a problem that the rotation of the baffle 21 is affected because the flange 213 interferes with the surrounding wall 204 in a process in which the baffle 21 rotates around the rotating shaft 22 from the first position to the second position, and ensure smoothness of the rotation of the rotating mechanism 2 between the first position and the second position.

The stop member 214 is located on the side of the surrounding wall 204 close to the airway chamber 11. In a specific implementation, the stop member 214 is arc-shaped, the surface of the stop member 214 close to the surrounding wall 204 is planar, and the limiting portion 2141 of the stop member 214 extending out of the second surface 212 abuts against the inner side surface of the surrounding wall 204, to further block the port of the first airflow channel Q1, to ensure that when the negative pressure in the airway chamber 11 is less than or equal to the preset threshold, the limiting portion 2141 of the stop member 214 of the baffle 21 can cooperate with the surrounding wall 204 of the mouthpiece 202, to better block the first airflow channel Q1, and avoid the problem that the drug powder is wasted because air enters the airway chamber 11 via the first airflow channel Q1 when the first airflow channel Q1 is not completely blocked. In addition, when the rotating mechanism 2 is reset from the second position to the first position, the limiting portion 2141 can also abut against the inner side surface of the surrounding wall 204, thereby limiting the rotating mechanism 2 to the first position.

In another implementation, the flange 213 and the stop member 214 may also be arranged in another shape, provided that the shape of the flange 213 is consistent with the cross sectional shape of the side wall of the port of the first airflow channel Q1, to better block the port of the first airflow channel Q1. The baffle 21 may not be provided with the flange 213 and the stop member 214, and the end surface of the baffle 21 away from the rotating shaft 22 may directly abut against the surrounding wall 204 of the mouthpiece 202 when the rotating mechanism 2 is at the first position, to block the first airflow channel Q1. Alternatively, only the flange 213 or only the stop member 214 may be provided. This may be designed based on requirements.

The dose protection mechanism 3 is further provided with a limiting member 32, and the outer side surface of the rotating shaft 22 is provided with a notch 221. When the rotating mechanism 2 is at the first position, the limiting member 32 abuts against the outer side surface of the rotating shaft 22, so that the dose protection mechanism 3 is limited to the third position by using the outer side surface of the rotating shaft 22. When the rotating mechanism 2 is at the second position, the limiting member 32 corresponds to the notch 221, to release the limit on the dose protection mechanism 3. To be specific, when the rotating mechanism 2 rotates to the second position, the limiting member 32 does not abut against the outer side surface of the rotating shaft 22, the notch 221 provided on the outer side surface of the rotating shaft 22 is rotated to a position corresponding to the limiting member 32, and the limiting member 32 is correspondingly located at a position of the notch 221, so that the rotating shaft 22 no longer limits the dose protection mechanism 3, facilitating movement of the dose protection mechanism 3.

In a specific implementation, referring to FIG. 3, FIG. 4A, FIG. 4B, FIG. 7, FIG. 8A, FIG. 8B, and FIG. 17, the dose protection mechanism 3 is slidably arranged on the support 1, the dose protection mechanism 3 further includes a body portion 33, and the plate-shaped portion 31 is connected to the side of the body portion 33 away from the elastic member 4. Specifically, the limiting member 32 is a protruding post obliquely arranged on the surface of the body portion 33. When the rotating mechanism 2 is at the first position, the free end of the protruding post abuts against the outer side surface of the rotating shaft 22, to limit the dose protection mechanism 3 to the third position. When the baffle 21 rotates around the rotating shaft 22, the notch 221 on the outer side surface of the rotating shaft 22 is rotated to a position of the free end of the protruding post, and the free end of the protruding post slides off the notch 221, to release the limit on the dose protection mechanism 3, so that the dose protection mechanism 3 can be driven by the elastic member 4 to slide from the third position to the fourth position.

In another implementation, the limiting member 32 of the dose protection mechanism 3 may also be arranged in another structure. For example, the limiting member 32 may be a groove or a notch provided on the body portion 33 of the dose protection mechanism 3. The notch 221 may not be provided on the outer side surface of the rotating shaft 22, and the protruding post is directly connected to the outer side surface of the rotating shaft 22. When the rotating mechanism 2 is at the first position, the free end of the protruding post abuts in a groove 222 of the body portion 33, so that the dose protection mechanism 3 is limited to the third position by using the protruding post arranged on the rotating shaft 22. When the baffle 21 rotates around the rotating shaft 22, the protruding post on the rotating shaft 22 rotates out of the groove 222 of the body portion 33, to release the limit on the dose protection mechanism 3, so that the dose protection mechanism 3 can move from the third position to the fourth position under the driving action of the elastic member 4. This is not limited in this application, provided that the limiting member 32 of the dose protection mechanism 3 is arranged in contact with the rotating mechanism 2, the dose protection mechanism 3 may be limited when the rotating mechanism 2 is at the first position, and the limit on the dose protection mechanism 3 may be released by the rotation of the rotating mechanism 2.

Further, the body portion 33 of the dose protection mechanism 3 is provided with an accommodation groove 331. Specifically, the accommodation groove 331 is provided on the surface of the body portion 33 close to the translation mechanism 5, and a protruding rod 23 is connected to the end of the rotating shaft 22. Specifically, the protruding rod 23 is connected to the end of the rotating shaft 22 close to the translation mechanism 5. Referring to FIG. 18, the translation mechanism 5 includes a support member 51 and a push rod 52 connected to each other, the support member 51 abuts against the protruding rod 23 on the rotating shaft 22, and the push rod 52 is at least partially arranged in the accommodation groove 331. When the translation mechanism 5 moves from the sixth position to the fifth position, the support member 51 pushes the protruding rod 23, to reset the rotating mechanism 2 from the second position to the first position, and the push rod 52 pushes the body portion 33, to reset the dose protection mechanism 3 from the fourth position to the third position.

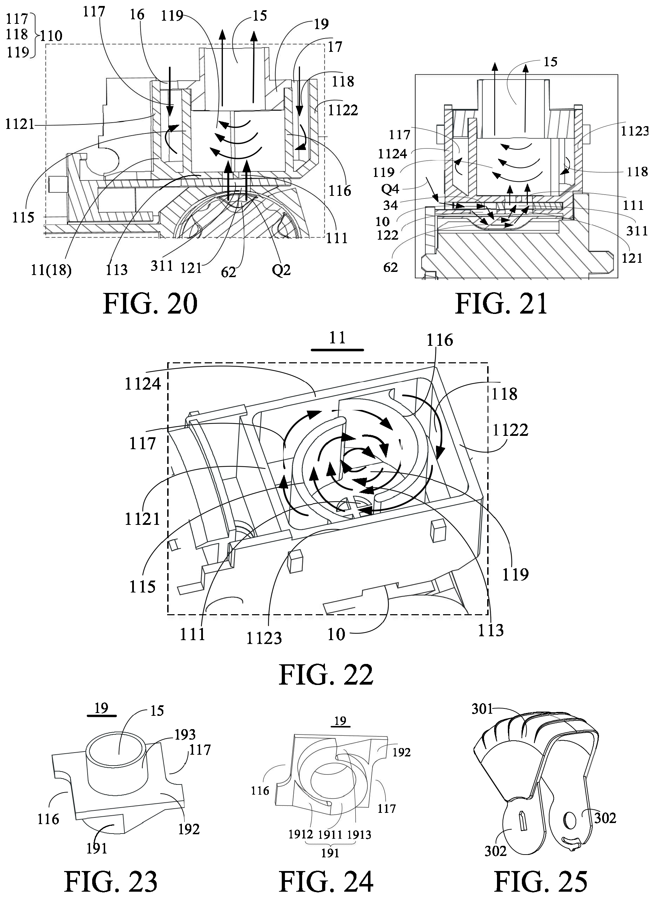

Referring to FIG. 25, FIG. 25 is a schematic structural diagram of a cover body of the dry powder inhaler provided in FIG. 1. Referring to FIG. 1, FIG. 2, FIG. 3, FIG. 6, FIG. 7, and FIG. 25, the cover body 300 is rotatable between a seventh position and an eighth position. When the cover body 300 is at the seventh position, that is, when the cover body 300 is in a closed state, the cover body 300 covers the mouthpiece 202 and the external air inlet 2011. When the cover body 300 rotates to the eighth position, that is, when the cover body 300 is in an open state, the cover body 300 exposes the mouthpiece 202 and the external air inlet 2011.

Specifically, the cover body 300 is approximately U-shaped, and includes an arc-shaped portion 301 and two rotating portions 302 connected to two ends of the arc-shaped portion 301. The two rotating portions 302 are symmetrically arranged. One of the rotating portions 302 is located on the side of the first housing 206 away from the second housing 207, and the rotating portion 302 is rotatably connected to a central position of the outer side surface of the first housing 206. The other of the rotating portions 302 is located on the side of the second housing 207 away from the first housing 206, and the rotating portion 302 is rotatably connected to a central position of the outer side surface of the second housing 207. The outer side surface of the first housing 206 and the outer side surface of the second housing 207 each are correspondingly provided with a first protruding portion 208 and a second protruding portion 209 spaced apart from each other. The cover body 300 may rotate between the first protruding portion 208 and the second protruding portion 209 in the peripheral direction of the housing assembly 200. The first protruding portion 208 and the second protruding portion 209 limit the cover body 300 to rotating between the seventh position and the eighth position. The first protruding portion 208 limits the cover body 300 to the seventh position, and the second protruding portion 209 limits the cover body 300 to the eighth position.

In a specific implementation, when the cover body 300 rotates from the eighth position to the seventh position, that is, in a process in which the cover body 300 rotates from the open state to the closed state, that is, in a process of closing a cover, the cover body 300 may drive the translation mechanism 5 to move from the sixth position to the fifth position, that is, drive the translation mechanism 5 to be reset to an initial position, so that the translation mechanism 5 drives the rotating mechanism 2 to be reset from the second position to the first position, and drives the dose protection mechanism 3 to be reset from the fourth position to the third position.

Specifically, referring to FIG. 18, the support member 51 includes a support portion 511 and a connection portion 512 connected to each other. The push rod 52 is connected to the surface of the side of the connection portion 512 close to the dose protection mechanism 3. The translation mechanism 5 further includes a projecting portion 53. The projecting portion 53 is connected to the end of the connection portion 512 away from the support portion 511. The support portion 511 is configured to abut against the protruding rod 23 on the rotating shaft 22, to push the protruding rod 23 to reset the rotating mechanism 2 from the second position to the first position when the translation mechanism 5 moves from the sixth position to the fifth position. In a process in which the cover body 300 rotates from the eighth position to the seventh position, the side surface of the rotating portion 302 of the cover body 300 abuts against the projecting portion 53, so that the cover body 300 pushes the projecting portion 53 of the translation mechanism 5, so that the translation mechanism 5 can move from the sixth position to the fifth position.

In a specific implementation, as shown in FIG. 25, FIG. 18, and FIG. 3, the surface of the projecting portion 53 close to the rotating portion 302 of the cover body 300 is an arc surface, and the surface of the rotating portion 302 of the cover body 300 close to the projecting portion 53 is a plane. When the cover body 300 is at the seventh position, that is, a position at which the cover body 300 is closed, the side surface of the rotating portion 302 of the cover body 300 abuts against the arc surface of the projecting portion 53, to limit the translation mechanism 5 to the fifth position. In a process in which the cover body 300 is opened, that is, in a process in which the cover body 300 rotates from the seventh position to the eighth position, the side surface of the rotating portion 302 of the cover body 300 is gradually separated from the arc surface of the projecting portion 53. After the cover body 300 rotates to the eighth position, that is, after the cover body 300 is completely opened, the side surface of the rotating portion 302 is completely separated from the arc surface of the projecting portion 53, to release the limit on the translation mechanism 5. In a process in which the cover body 300 is closed, that is, in a process in which the cover body 300 rotates from the eighth position to the seventh position, the side surface of the rotating portion 302 of the cover body 300 gradually abuts against the arc surface of the projecting portion 53. In a process in which the rotating portion 302 of the cover body 300 rotates in the peripheral direction of the housing 201, the side surface of the rotating portion 302 abuts against a different position of the arc surface of the projecting portion 53, and the side surface of the rotating portion 302 pushes the projecting portion 53 to move in the horizontal direction in the second direction A2, so that the translation mechanism 5 is reset from the sixth position to the fifth position. After the cover body 300 is reset to the seventh position, that is, after the cover is completely closed, the side surface of the rotating portion 302 of the cover body 300 limits the translation mechanism 5 to the fifth position again.

When the cover body 300 is at the seventh position, the cover body 300 covers the mouthpiece 202, and the user cannot inhale at the mouthpiece 202. The cover body 300 limits the translation mechanism 5 to the fifth position, the translation mechanism 5 limits the rotating mechanism 2 to the first position, and the rotating mechanism 2 limits the dose protection mechanism 3 to the third position. In this case, the mouthpiece 202 is covered, and even if the negative pressure in the airway chamber 11 is greater than the preset threshold, the rotating mechanism 2 is limited to the first position only and cannot rotate. When the cover body 300 is at the eighth position, the mouthpiece 202 is exposed, and the cover body 300 releases the limit on the translation mechanism 5, facilitating movement of the translation mechanism 5 from the fifth position to the sixth position.

In an implementation, the cover body 300 may be connected to the translation mechanism 5. The translation mechanism 5 may be driven to directly slide between the fifth position and the sixth position by rotation of the cover body 300 between the seventh position and the eighth position. In other words, when the cover body 300 moves from the seventh position to the eighth position, the cover body 300 directly drives the translation mechanism 5 to move from the fifth position to the sixth position.

In an implementation, as shown in FIG. 3, an elastic arm 13 may be arranged on the support 1. When the translation mechanism 5 is at the fifth position, the elastic arm 13 abuts against the translation mechanism 5, so that the elastic arm 13 is in a compressed state. When the cover body 300 is at the eighth position, that is, after the cover body 300 releases the limit on the translation mechanism 5, the elastic arm 13 drives the translation mechanism 5 to slide from the fifth position to the sixth position. In this case, the support portion 511 of the translation mechanism 5 no longer abuts against the protruding rod 23 of the rotating mechanism 2, that is, the support portion 511 no longer interferes with movement of the protruding rod 23 of the rotating mechanism 2, to avoid the problem of an abnormal breath triggering function caused because the support portion 511 of the translation mechanism 5 blocks normal rotation of the rotating mechanism 2 in a breath triggering process after the cover body 300 is completely opened.

In an implementation, the translation mechanism 5 may be directly driven to slide from the fifth position to the sixth position when the rotating mechanism 2 rotates from the first position to the second position. Specifically, after the cover body 300 releases the limit on the translation mechanism 5, the protruding rod 23 connected to the rotating shaft 22 of the rotating mechanism 2 may abut against the support portion 511 of the translation mechanism 5, and based on the rotation of the baffle 21 about the rotating shaft 22, the protruding rod 23 pushes the support portion 511 to move, to drive the translation mechanism 5 to slide from the fifth position to the sixth position.

In an implementation, the translation mechanism 5 may be directly driven to slide from the fifth position to the sixth position when the dose protection mechanism 3 moves from the third position to the fourth position. Specifically, after the cover body 300 releases the limit on the translation mechanism 5, the baffle 21 of the rotating mechanism 2 rotates around the rotating shaft 22 to release the limit on the dose protection mechanism 3, so that the dose protection mechanism 3 moves from the third position to the fourth position under the driving action of the elastic member 4. The push rod 52 of the translation mechanism 5 abuts against the side wall of the accommodation groove 331 of the dose protection mechanism 3, and the dose protection mechanism 3 moves to drive the push rod 52 of the translation mechanism 5 to move, so that the translation mechanism 5 slides from the fifth position to the sixth position.

In another implementation, the translation mechanism 5 may move from the sixth position to the fifth position without driving of the rotation of the cover body 300 from the eighth position to the seventh position. For example, a separate reset mechanism may be arranged, and the reset mechanism is connected to the translation mechanism 5, so that the reset mechanism directly drives the translation mechanism 5 to be reset from the sixth position to the fifth position. Alternatively, the translation mechanism 5 may be limited to the fifth position without using the cover body 300. For example, a separate limiting mechanism may be arranged, to limit the translation mechanism 5 to the fifth position.

In a specific implementation, as shown in FIG. 15 and FIG. 16, the side surface of the end of the rotating shaft 22 is provided with a groove 222, the end of the protruding rod 23 is connected to the side wall of the groove 222, and an included angle is provided between the protruding rod 23 and the baffle 21. Specifically, the protruding rod 23 is located on the side of the baffle 21 facing away from the first surface 211. It may be understood that, the side surface of the end of the rotating shaft 22 is provided with the groove 222, and the end of the protruding rod 23 is directly connected to the side wall of the groove 222. In a process in which the support member 51 of the translation mechanism 5 abuts against the protruding rod 23 to drive the protruding rod 23 to rotate around the rotating shaft 22 and reset the rotating mechanism 2 to the first position, the protruding rod 23 is not likely to be broken under the action of the support member 51, and rotation efficiency of the protruding rod 23 and the rotating shaft 22 under the action of the support member 51 is higher. In addition, the included angle is provided between the protruding rod 23 and the baffle 21, that is, the protruding rod 23 is arranged obliquely to the baffle 21, and the protruding rod 23 is located on the side of the baffle 21 facing away from the first surface 211. When the translation mechanism 5 slides from the sixth position to the fifth position, the protruding rod 23 is more likely to abut against the support member 51 of the translation mechanism 5, so that the support member 51 of the translation mechanism 5 better applies a force to the protruding rod 23, the protruding rod 23 rotates around the rotating shaft 22 to drive the baffle 21 of the rotating mechanism 2 to rotate around the rotating shaft 22 more smoothly, and the translation mechanism 5 drives the rotating mechanism 2 to be reset from the second position to the first position more efficiently. Preferably, the included angle between the protruding rod 23 and the baffle 21 may be set in a range of 15° to 30°, to more effectively improve resetting efficiency of the rotating mechanism 2.

In another implementation, the side surface of the end of the rotating shaft 22 may not be provided with the groove 222, and the end of the protruding rod 23 is directly connected to the end surface of the end of the rotating shaft 22 close to the translation mechanism 5 or connected to the outer side surface of the rotating shaft 22. The translation mechanism 5 drives the protruding rod 23 of the rotating mechanism 2 to rotate, to drive the rotating mechanism 2 to be reset from the second position to the first position.

Referring to FIG. 3 to FIG. 14, specifically, the airway chamber 11 and the dosage chamber 12 are spaced apart from each other in the first direction A1, the first airflow channel Q1 and the rotating mechanism 2 are arranged on the side of the airway chamber 11 in the second direction A2, and the dose protection mechanism 3 slides between the third position and the fourth position in the second direction A2. The shaft direction of the rotating shaft 22 of the rotating mechanism 2 is the third direction A3, and the translation mechanism 5 is arranged on the side of the dose protection mechanism 3 in the third direction A3. The first direction A1, the second direction A2, and the third direction A3 are perpendicular to each other.

In some implementations, before the breath triggering device 100 is triggered, when the negative pressure in the airway chamber 11 is less than or equal to the preset threshold, that is, before the first airflow channel Q1 is opened, the airway chamber 11 is further in communication with the external air via a third airflow channel Q3, to ensure consistency of inhalation resistance in the airway chamber 11 at all stages. Specifically, as shown in FIG. 17, the plate-shaped portion 31 of the dose protection mechanism 3 is further provided with a second communication hole 312, and the second communication hole 312 and the first communication hole 311 are spaced apart from each other. In a specific implementation, the second communication hole 312 and the first communication hole 311 are aligned with each other and spaced apart in the second direction A2. The second communication hole 312 is the part of the third airflow channel Q3. Before the breath triggering device 100 is triggered, when the dose protection mechanism 3 is at the third position, the third airflow channel Q3 is opened, and the second communication hole 312 is in communication with the airway chamber 11 and the external air, to balance inhalation resistance in the airway chamber 11, to avoid the problem that user experience is affected because inhalation resistance in the airway chamber 11 is inconsistent. After the breath triggering device 100 is triggered, when the negative pressure in the airway chamber 11 is greater than the preset threshold, and when the dose protection mechanism 3 is at the fourth position, the second communication hole 312 is not in communication with the airway chamber 11, the third airflow channel Q3 is blocked, and the first airflow channel Q1 is opened. In this case, a communication manner of the airflow channel of the dry powder inhaler 800 is changed, and the airway chamber 11 is in communication with the external air via the first airflow channel Q1. The airway chamber 11 is in communication with the external air via different airflow channels in different time periods, which ensures consistency of inhalation resistance in the airway chamber 11 in each time, improves user compliance, and also facilitates improving the deaggregation effect on the drug powder.

Specifically, referring to FIG. 4A, FIG. 4B, FIG. 8A, FIG. 8B, and FIG. 13, the support 1 is provided with a third communication hole 14, and the third communication hole 14 and the airway chamber 11 are spaced apart from each other. In a specific implementation, the third communication hole 14 and the second powder discharge hole 111 are spaced apart from each other in the second direction A2, the third communication hole 14 is located on the side of the airway chamber 11 close to the rotating mechanism 2, the third communication hole 14 is in communication with the airway chamber 11 via a gap between the airway chamber 11 and the rotating mechanism 2, the second communication hole 312 is provided on the plate-shaped portion 31 of the dose protection mechanism 3, and the end of the second communication hole 312 is in communication with the external air. Specifically, the end of the second communication hole 312 is in communication with the external air entering the inside of the housing assembly 200.

As shown in FIG. 4A and FIG. 4B, before the breath triggering device 100 is triggered, the rotating mechanism 2 is at the first position and limits the dose protection mechanism 3 to the third position, the port of the first airflow channel Q1 is blocked, and the second communication hole 312 on the plate-shaped portion 31 of the dose protection mechanism 3, the third communication hole 14 on the support 1, and the gap between the airway chamber 11 and the rotating mechanism 2 are in communication sequentially to form the third airflow channel Q3. Before the breath triggering device 100 is triggered, when the dose protection mechanism 3 is at the third position, the second communication hole 312 on the dose protection mechanism 3 and the third communication hole 14 on the support 1 are aligned with each other and in communication with each other, that is, the third airflow channel Q3 is opened, so that the second communication hole 312 is in communication with the airway chamber 11 and the external air, and the airway chamber 11 is in communication with the external air via the third airflow channel Q3, to balance inhalation resistance in the airway chamber 11.

When the dose protection mechanism 3 moves to the fourth position in the second direction A2, that is, after the breath triggering device 100 is triggered, the second communication hole 312 is staggered with and cannot be in communication with the third communication hole 14 on the support 1, so that the second communication hole 312 is not in communication with the airway chamber 11. The plate-shaped portion 31 of the dose protection mechanism 3 blocks the third communication hole 14 on the support 1, so that the third airflow channel Q3 is blocked, and the airway chamber 11 cannot be in communication with the external air via the third airflow channel Q3. In this case, the rotating mechanism 2 has already rotated and opens the port of the first airflow channel Q1, and the airway chamber 11 is converted to be in communication with the external air via the port of the first airflow channel Q1. In addition, after the breath triggering device 100 is triggered, the first powder discharge hole 121, the first communication hole 311, and the second powder discharge hole 111 are in alignment and communication sequentially, that is, the second airflow channel Q2 is opened, so that the user can inhale the drug powder from the medicament accommodating portion 62 in the dosage chamber 12 into the airway chamber 11 during inhalation, to ensure that the flow rate of the airflow is sufficiently large to better deaggregate the drug powder. In another implementation, the support 1 may not be provided with the third communication hole 14, and when the dose protection mechanism 3 is at the third position, the second communication hole 312 is in communication with the airway chamber 11 and the external air in another manner.

Through the arrangement of the breath triggering device 100, an action of the breath triggering device 100 is triggered only when the flow rate of the airflow during inhalation of the user is sufficiently greater than the working threshold and the negative pressure in the airway chamber 11 is greater than the preset threshold, so that the second airflow channel Q2 is in communication, and the drug powder in the medicament accommodating portion 62 of the base tape 61 in the dosage chamber 12 enters the airway chamber with the airflow and is deaggregated better, thereby improving the utilization of the drug powder, and improving an emptying rate of the drug powder in the medicament accommodating portion 62. In addition, when the flow rate of the airflow during inhalation of the user is less than or equal to the working threshold and the negative pressure in the airway chamber 11 is less than or equal to the preset threshold, the breath triggering device 100 is not triggered, the second airflow channel Q2 is blocked by the plate-shaped portion 31 of the dose protection mechanism 3, the drug powder in the medicament accommodating portion 62 in the dosage chamber 12 is blocked in the dosage chamber 12 by the plate-shaped portion 31, and the drug powder does not enter the airway chamber 11, to avoid the problems of a poor deaggregation effect on the drug powder and low utilization of the drug powder caused due to insufficient airflow during inhalation of the user. In addition, the plate-shaped portion 31 blocks the drug powder in the medicament accommodating portion 62, which also avoids problems such as pollution of the drug powder and leakage of the drug powder when the user does not inhale or when the airflow is insufficient during inhalation of the user.

In this application, before and after the breath triggering device 100 is triggered, positions and motion states of the rotating mechanism 2 and the dose protection mechanism 3 are changed, and the communication manner of the airflow channel of the dry powder inhaler 800 is also changed. Specifically, when the negative pressure in the airway chamber 11 is less than or equal to the preset threshold, the third airflow channel Q3 is opened, the first airflow channel Q1 is blocked, and the third airflow channel Q3 communicates the airway chamber 11 with the external air. When the negative pressure in the airway chamber 11 is greater than the preset threshold, the first airflow channel Q1 is opened, the third airflow channel Q3 is blocked, and the first airflow channel Q1 communicates the airway chamber 11 with the external air. Through the arrangement of the breath triggering device 100, communication of different airflow channels of the dry powder inhaler 800 is implemented.

Referring to FIG. 19 to FIG. 24, FIG. 19 is a schematic structural diagram of an embodiment of an airway structure of a dry powder inhaler according to this application; FIG. 20 is a cross sectional schematic view of the airway structure provided in FIG. 19; FIG. 21 is another cross sectional schematic view of the airway structure provided in FIG. 19; FIG. 22 is a schematic structural diagram of an airway chamber of the airway structure provided in FIG. 19; FIG. 23 is a top schematic structural diagram of a manifold of the airway structure provided in FIG. 19; FIG. 24 is a bottom schematic structural diagram of the manifold provided in FIG. 23.

Referring to FIG. 4A, FIG. 4B, FIG. 6, FIG. 8A, FIG. 8B, and FIG. 19 to FIG. 24, in an embodiment, the dry powder inhaler 800 further includes an airway structure. The airway structure includes the foregoing structures such as the airway chamber 11, the first airflow channel Q1, the third airflow channel Q3, the breath triggering device 100, and the external air inlet 2011 provided on the housing 201. The state of the breath triggering device 100 is determined based on values of the negative pressure in the airway chamber 11 and the preset threshold, so that in two different states before and after the breath triggering device 100 is triggered, the dry powder inhaler 800 has different communication manners of airflow channels. This ensures consistency of inhalation resistance at all stages, and is also more beneficial to deaggregation on the drug powder.

In another embodiment, another triggering mechanism different from the breath triggering device 100 may also be arranged, to implement communication or switching of different airflow channels of the dry powder inhaler 800 based on an action of the triggering mechanism, so that in different states of the triggering mechanism, the dry powder inhaler 800 has different communication manners of airflow channels, to ensure consistency of inhalation resistance of the dry powder inhaler 800 at all stages.

Referring to FIG. 11 to FIG. 14 and FIG. 19 to FIG. 23, the airway structure includes the support 1, and the support 1 is provided with the foregoing airway chamber 11. The airway chamber 11 includes the annular side wall 112, the bottom wall 113, and the top wall 114. The bottom wall 113 of the airway chamber 11 is provided with the second powder discharge hole 111. A first fin 115 and a second fin 116 are arranged in the airway chamber 11. The first fin 115 and the second fin 116 are spaced apart from each other. The first fin 115 cooperates with the annular side wall 112 to form a first airway portion 117. The second fin 116 cooperates with the annular side wall 112 to form a second airway portion 118. The first fin 115 and the second fin 116 cooperates with the annular side wall 112 to form a mixed airway portion 119. The first airway portion 117 and the second airway portion 118 each are in communication with the mixed airway portion 119. The first airway portion 117, the second airway portion 118, and the mixed airway portion 119 jointly form a vortex airway 110. Specifically, a vortex flow is formed inside the mixed airway portion 119, and the vortex airway 110 is in a spiral shape. The top wall 114 of the airway chamber 11 is provided with an air outlet hole 15 and an air inlet spaced from each other. Specifically, the air inlet includes a first air inlet 16 and a second air inlet 17. The first air inlet 16 and the second air inlet 17 are respectively provided on two sides of the air outlet hole 15. The air outlet hole 15 is directly in communication with the mixed airway portion 119. The first air inlet 16 communicates the external air with the first airway portion 117, and the second air inlet 17 communicates the external air with the second airway portion 118.

It may be understood that, the foregoing airway structure is arranged in the dry powder inhaler 800, the first fin 115 and the second fin 116 are arranged in the airway chamber 11, to form the first airway portion 117, the second airway portion 118, and the mixed airway portion 119, and the first airway portion 117 and the second airway portion 118 each are in communication with the mixed airway portion 119 to form the vortex airway 110 in the spiral shape. The top wall 114 of the airway chamber 11 are provided with two air inlets: the first air inlet 16 and the second air inlet 17, the first air inlet 16 is in communication with the first airway portion 117, and the second air inlet 17 is in communication with the second airway portion 118. The two air inlets are provided, so that external airflow can more efficiently enter the vortex airway 110 from the top wall 114 of the airway chamber 11 when the user inhales at the mouthpiece 202. This is more beneficial to deaggregation on the drug powder entering the airway chamber 11. In addition, the first airway portion 117 and the second airway portion 118 each are in communication with the mixed airway portion 119 to form the vortex airway 110. During inhalation of the user, two tangent airflows are formed between air entering the mixed airway portion 119 from the first airway portion 117 and air entering the mixed airway portion 119 from the second airway portion 118 form two tangential airflows. The two tangential airflows aggregate to form a vortex flow in the mixed airway portion 119, which is more beneficial to disaggregation on the drug powder in the mixed airway portion 119, and is more beneficial for disaggregated drug powder to be inhaled by the user via the air outlet hole 15 and the mouthpiece 202, thereby improving the disaggregation effect of the drug powder. The drug powder in the mixed airway portion 119 have a higher emptying rate under the action of a swirl, thereby reducing attachment of the drug powder to the side wall and the bottom wall 113 of the mixed airway portion 119, improving the utilization of the drug powder during each inhalation, and reducing waste of the drug powder.

Referring to FIG. 22, in a specific implementation, the first fin 115 and the second fin 116 each are arc-shaped. The concave surfaces of the first fin 115 and the second fin 116 are arranged opposite to each other, to cooperate to form the mixed airway portion 119. The convex surface of the first fin 115 faces the annular side wall 112 and is spaced apart from the annular side wall 112 to form the first airway portion 117. The convex surface of the second fin 116 faces the annular side wall 112 and is spaced apart from the annular side wall 112 to form the second airway portion 118. The first air inlet 16 is provided corresponding to the first airway portion 117, and the second air inlet 17 is provided corresponding to the second airway portion 118. Specifically, the first air inlet 16 is provided corresponding to the end of the first airway portion 117 away from the mixed airway portion 119, so that air entering the first airway portion 117 from the first air inlet 16 can flow through the entire first airway portion 117 and then enter the mixed airway portion 119. The second air inlet 17 is provided corresponding to the end of the second airway portion 118 away from the mixed airway portion 119, so that air entering the second airway portion 118 from the second air inlet 17 can flow through the entire second airway portion 118 and then enter the mixed airway portion 119. This improves flow rates of two tangent airflows entering the mixed airway portion 119 from the first airway portion 117 and the second airway portion 118 respectively, and further improving the deaggregation effect of the drug powder and the emptying rate and the utilization of the drug powder. The second powder discharge hole 111 is provided corresponding to the center of the vortex airway 110. Specifically, the second powder discharge hole 111 corresponds to a central position of the mixed airway portion 119 and is provided on the bottom wall 113 of the airway chamber 11, so that air entering the vortex airway 110 can further perform more even and more efficient deaggregation on the drug powder entering the mixed airway portion 119 from the second powder discharge hole 111.

Specifically, the first fin 115 and the second fin 116 each extend from the bottom wall 113 of the airway chamber 11 to the top wall 114 of the airway chamber 11. In a specific implementation, in the first direction A1, the bottom ends of the first fin 115 and the second fin 116 are connected to the bottom wall 113 of the airway chamber 11, and the top ends of the first fin 115 and the second fin 116 are arranged in contact with the top wall 114 of the airway chamber 11. The annular side wall 112 is in a shape of a rectangular ring. The annular side wall 112 includes the first side wall 1121 and the second side wall 1122 arranged opposite to each other, and the third side wall 1123 and the fourth side wall 1124 arranged opposite to each other. In the extension direction of the arc surface, the end of the first fin 115 is connected to the third side wall 1123, and the other end of the first fin 115 extends toward the fourth side wall 1124 and is spaced apart from the fourth side wall 1124. The first fin 115 cooperates with the first side wall 1121 and the part of the fourth side wall 1124 to form the first airway portion 117. The first airway portion 117 is in communication with the mixed airway portion 119 via a gap between the first fin 115 and the fourth side wall 1124. The end of the second fin 116 is connected to the fourth side wall 1124, and the other end of the second fin 116 extends toward the third side wall 1123 and is spaced apart from the third side wall 1123. The second fin 116 cooperates with the second side wall 1122 and the part of the third side wall 1123 to form the second airway portion 118. The second airway portion 118 is in communication with the mixed airway portion 119 via a gap between the second fin 116 and the third side wall 1123. In this way, the second airway portion 118 and the first airway portion 117 enter the mixed airway portion 119 from opposite positions, to form swirling airflow.

As shown in FIG. 19 and FIG. 22, in a specific implementation, the first fin 115 and the second fin 116 are centrally and symmetrically arranged, and the first air inlet 16 and the second air inlet 17 are centrally and symmetrically arranged. This is more beneficial to forming of the vortex airway 110, thereby improving a deaggregation effect of the swirling airflow on the drug powder.

In another implementation, the first fin 115 and the second fin 116 may also be arranged in another shape. For example, the first fin 115 and the second fin 116 each may be arranged as folded surfaces. The concave surfaces of the first fin 115 and the second fin 116 are arranged opposite to each other to cooperate to form the mixed airway portion 119. The convex surface of the first fin 115 cooperates with the first side wall 1121 and the part of the fourth side wall 1124 to form the first airway portion 117. The convex surface of the second fin 116 cooperates with the second side wall 1122 and the part of the third side wall 1123 to form the second airway portion 118. The first airway portion 117 and the second airway portion 118 each are in communication with the mixed airway portion 119 to form the vortex airway 110. The first fin 115 and the second fin 116 may alternatively be in any shape like a curved surface. Alternatively, the first fin 115 may not be connected to the third side wall 1123, and the second fin 116 may not be connected to the fourth side wall 1124. For example, the first fin 115 is spaced apart from each of the third side wall 1123 and the fourth side wall 1124, and the second fin 116 is spaced apart from each of the third side wall 1123 and the fourth side wall 1124. This is not limited in this application, provided that the first fin 115 and the second fin 116 can cooperate with the annular side wall 112 of the airway chamber 11 to form the first airway portion 117 and the second airway portion 118, and the first fin 115 and the second fin 116 cooperate with each other to form the mixed airway portion 119 to form the vortex airway 110.

In a specific implementation, referring to FIG. 19 to FIG. 24, the support 1 includes a support body 18 and a manifold 19. The surface of the support body 18 close to the mouthpiece 202 is provided with a gas collecting groove (not marked in the figure). The side wall of the gas collecting groove is used as the annular side wall 112 of the airway chamber 11. The bottom wall 113 of the gas collecting groove is used as the bottom wall 113 of the airway chamber 11. The bottom ends of the first fin 115 and the second fin 116 are connected to the bottom wall 113 of the gas collecting groove. The first airway portion 117, the second airway portion 118, and the mixed airway portion 119 are all formed in the gas collecting groove. The manifold 19 is arranged on the side of the support body 18 and covers the gas collecting groove. The manifold 19 is used as the top wall 114 of the airway chamber 11. The first air inlet 16 and the second air inlet 17 are provided on the manifold 19. The top ends of the first fin 115 and the second fin 116 each extend to be in contact with the manifold 19, to avoid the problem: airflows entering the gas collecting groove from the first air inlet 16 and the second air inlet 17 directly enter the mixed airway portion 119 via gaps between the top ends of the first fin 115 and the second fin 116, and the manifold 19, and do not flow or only flow partially through the first airway portion 117 and the second airway portion 118, affecting forming of the swirling airflow in the vortex airway 110, causing a poor deaggregation effect on the drug powder in the mixed airway portion 119 and a low emptying rate of the drug powder.

Specifically, as shown in FIG. 23 and FIG. 24, the manifold 19 includes an insertion portion 191, a covering portion 192, and a communicating portion 193 connected to each other. The insertion portion 191 is located on the side of the covering portion 192 away from the communicating portion 193. The insertion portion 191 is in a spiral shape. The insertion portion 191 is inserted in the vortex airway 110. The covering portion 192 covers the top end of the support body 18 and covers the gas collecting groove. The first air inlet 16 and the second air inlet 17 are both provided on the covering portion 192. The air outlet hole 15 sequentially extends through the communicating portion 193, the covering portion 192, and the insertion portion 191, and is in communication with the mixed airway portion 119.

Specifically, the covering portion 192 is in a rectangular plate-shaped structure. Four sides of the covering portion 192 respectively correspondingly abut against four side walls of the airway chamber 11. The first air inlet 16 and the second air inlet 17 are notches provided on the covering portion 192. The first air inlet 16 is provided on the surface of the covering portion 192 close to the first side wall 1121, and extends from the surface of the covering portion 192 close to the third side wall 1123 to the surface of the covering portion 192 close to the fourth side wall 1124. The first air inlet 16 and the fourth side wall 1124 are spaced apart from each other in the third direction A3. The second air inlet 17 is provided on the surface of the covering portion 192 close to the second side wall 1122, and extends from the surface of the covering portion 192 close to the fourth side wall 1124 to the surface of the covering portion 192 close to the third side wall 1123. The second air inlet 17 and the third side wall 1123 are spaced apart from each other in the third direction A3. Preferably, the first air inlet 16 and the second air inlet 17 are centrally and symmetrically provided.

In another implementation, the first air inlet 16 and the second air inlet 17 each may alternatively in any structure like a circular hole or a square hole provided on the covering portion 192. The shapes of the first air inlet 16 and the second air inlet 17 may be the same or different. The first air inlet 16 and the second air inlet 17 may alternatively be provided on other positions of the covering portion 192. The first air inlet 16 and the second air inlet 17 may not be centrally and symmetrically provided, provided that the first air inlet 16 may be in communication with the first airway portion 117 and the second air inlet 17 may be in communication with the second airway portion 118.

As shown in FIG. 23, the communicating portion 193 of the manifold 19 is in a circular tube shape, and the end of the communicating portion 193 away from the insertion portion 191 is inserted in the mouthpiece 202, so that the mouthpiece 202 is in communication with the mixed airway portion 119 via the air outlet hole 15 of the manifold 19, which is beneficial for the user to inhale the drug powder in the mixed airway portion 119 at the mouthpiece 202.

As shown in FIG. 24, the insertion portion 191 is in a spiral shape. Specifically, the insertion portion 191 includes an annular insertion segment 1911, and a first insertion segment 1912 and a second insertion segment 1913 that are connected to the annular insertion segment 1911 and spaced apart from each other. Preferably, both the first insertion segment 1912 and the second insertion segment 1913 are spaced apart from parts of the outer side surface of the annular insertion segment 1911. The first insertion segment 1912 is arranged corresponding to the gap between the first fin 115 and the fourth side wall 1124. The second insertion segment 1913 is arranged corresponding to the gap between the second fin 116 and the third side wall 1123. The annular insertion segment 1911 is inserted in the mixed airway portion 119. The first insertion segment 1912 is inserted in the gap between the first fin 115 and the fourth side wall 1124. The second insertion segment 1913 is inserted in the gap between the second fin 116 and the third side wall 1123. The end of the first fin 115 close to the fourth side wall 1124 is inserted between the first insertion segment 1912 and the annular insertion segment 1911. The end of the second fin 116 close to the third side wall 1123 is inserted between the second insertion segment 1913 and the annular insertion segment 1911. This facilitates stable assembly and combination of the manifold 19 and the support body 18, and makes it easier for the swirling airflow to be formed in the vortex airway 110.

Preferably, as shown in FIG. 24, the surface of the first insertion segment 1912 and the surface of the second insertion segment 1913 that are of the manifold 19 and away from the covering portion 192 are inclined surfaces. From the end connected to the annular insertion segment 1911 to the end away from the annular insertion segment 1911, the thickness of the first insertion segment 1912 and the thickness of the second insertion segment 1913 in the first direction A1 gradually decrease. It may be understood that, the surface of the first insertion segment 1912 and the surface of the second insertion segment 1913 that are away from the covering portion 192 are both arranged as the inclined surfaces, to have a particular guide effect for airflow entering via the first air inlet 16 and the second air inlet 17. The inclined surfaces are more beneficial to forming of the swirling airflow in the vortex airway 110, to facilitate improvement of the deaggregation effect on the drug powder.

Specifically, the outer side surface of the first insertion segment 1912 is planar and abuts against the fourth side wall 1124; the inner side surface of the first insertion segment 1912 is an arc surface and abuts against the part of the outer side surface of the first fin 115; the outer side surface of the second insertion segment 1913 is a plane and abuts against the third side wall 1123; and an inner side surface of the second insertion segment 1913 is an arc surface and abuts against the part of the outer side surface of the second fin 116. More preferably, as shown in FIG. 24, in the second direction A2, the first air inlet 16 and the first insertion segment 1912 are spaced apart from each other, and/or the second air inlet 17 and the second insertion segment 1913 are spaced apart from each other, to avoid the problem: airflow entering the first airway portion 117 from the first air inlet 16 directly enters the mixed airway portion 119 via a connection position between the second airway portion 118 and the mixed airway portion 119, or airflow entering the second airway portion 118 from the first air inlet 16 directly enters the mixed airway portion 119 via a connection position between the second airway portion 118 and the mixed airway portion 119, affecting forming of the swirling airflow, and then affecting the deaggregation effect on the drug powder.

In another implementation, the manifold 19 may alternatively be integrally formed with the support body 18, or the manifold 19 may not be arranged. The top wall 114 of the airway chamber 11 is formed on the support body 18. The top ends of the first fin 115 and the second fin 116 may be directly connected to the top wall 114 of the airway chamber 11. The air outlet hole 15 is provided on the top wall 114 of the airway chamber 11 to communicate the mouthpiece 202 with the mixed airway portion 119. The first fin 115 and the second fin 116 may be integrally formed with the support 1.

The dosage chamber 12 is for accommodating the drug tape 6. Specifically, the drug tape 6 includes the base tape 61 and a cover tape (not shown in the figure). The base tape 61 is provided with the medicament accommodating portion 62 for accommodating a powdered drug. The cover tape is adhered to the surface of the base tape 61 and covers the medicament accommodating portion 62. During use, the cover tape is peeled from the base tape 61, and the dosage chamber 12 is for accommodating the base tape 61 from which the cover tape is peeled, so that the medicament accommodating portion 62 is exposed, facilitating inhalation of the user on the drug powder in the medicament accommodating portion 62.

In some implementations, the airway structure further includes an air inlet channel Q4 independent of the vortex airway 110. The air inlet channel Q4 communicates the external air with the second powder discharge hole 111 on the bottom wall 113 of the airway chamber 11, and passes through the first powder discharge hole 121 on the top wall 114 of the dosage chamber 12. During inhalation of the user, the medicament accommodating portion 62 of the drug tape 6 is delivered to a position corresponding to the first powder discharge hole 121 of the dosage chamber 12, that is, the first powder discharge hole 121 is in communication with the medicament accommodating portion 62. It may be understood that, the air inlet channel Q4 independent of the vortex airway 110 is arranged in the airway structure. The air inlet channel Q4 is in communication with the external air, and needs to pass through the first powder discharge hole 121 on the top wall 114 of the dosage chamber 12 and then is in communication with the second powder discharge hole 111. During inhalation of the user, the air inlet channel Q4 may guide external airflow to the first powder discharge hole 121, and the airflow may carry the drug powder in the medicament accommodating portion 62 aligned with the first powder discharge hole 121 to enter the second powder discharge hole 111, so that the drug powder is inhaled by the user via the vortex airway 110 and the mouthpiece 202. In other words, when the airflow passes through the first powder discharge hole 121, the airflow passes through the above of the medicament accommodating portion 62, and the drug powder in the medicament accommodating portion 62 is more easily carried into the vortex airway 110 by the airflow. In this way, the drug powder can be discharged from the medicament accommodating portion 62, thereby effectively resolving the problem that the drug powder is incompletely discharged, and improving the utilization of the drug powder.

In an implementation, the top wall 114 of the dosage chamber 12 is further provided with a vent hole 122 spaced apart from the first powder discharge hole 121. The vent hole 122 is staggered with the second powder discharge hole 111. The air inlet channel Q4 includes the vent hole 122, the medicament accommodating portion 62 in the dosage chamber 12, and the first powder discharge hole 121 that are in communication sequentially. The end of the air inlet channel Q4 is in communication with the external air, and the other end of the air inlet channel Q4 is in communication with the second powder discharge hole 111. During inhalation of the user, the air inlet channel Q4 sequentially flows through the vent hole 122, the medicament accommodating portion 62 in the dosage chamber 12, and the first powder discharge hole 121, and then reaches the second powder discharge hole 111. It may be understood that, the air entering the air inlet channel Q4 first flows through the vent hole 122 before flowing through the first powder discharge hole 121. Because the vent hole 122 and the first powder discharge hole 121 are spaced apart from each other, when the air flows from the vent hole 122 to the second powder discharge hole 111, the air passes through the inside of the dosage chamber 12. Specifically, during inhalation of the user, the medicament accommodating portion 62 of the drug tape 6 is delivered to a position corresponding to the vent hole 122 and the first powder discharge hole 121. Air flows through the above of the medicament accommodating portion 62 in the dosage chamber 12, and then flows through the first powder discharge hole 121. Airflow flowing through the above of the medicament accommodating portion 62 carries the drug powder in the medicament accommodating portion 62, enters the first powder discharge hole 121, and then flows through the vortex airway 110, so that the drug powder is inhaled by the user at the mouthpiece 202. In comparison with that the drug powder in the medicament accommodating portion 62 is draw into the vortex airway 110 via only the second airflow channel Q2, the independent air inlet channel Q4 is arranged, so that the airflow can flow through the inside of the medicament accommodating portion 62, which is more beneficial to improving the emptying rate and the utilization of the drug powder, thereby avoiding waste caused by incomplete discharge of the drug powder.

Specifically, as shown in FIG. 14 and FIG. 21, the support 1 is provided with a third air inlet 10. The third air inlet 10 is provided corresponding to a position between the top wall 114 of the dosage chamber 12 and the bottom wall 113 of the airway chamber 11. The third air inlet 10 communicates the external air with the vent hole 122. The vent hole 122 and the first powder discharge hole 121 are both for being in communication with the medicament accommodating portion 62 of the drug tape 6 during inhalation of the user, so that the third air inlet 10, the vent hole 122, the medicament accommodating portion 62, and the first powder discharge hole 121 are in communication with each other sequentially to form the air inlet channel Q4. In other words, the third air inlet 10 is the port of the air inlet channel Q4, and external air enters the air inlet channel Q4 via the third air inlet 10.

In a specific implementation, during inhalation of the user, the medicament accommodating portion 62 is delivered to the position corresponding to the vent hole 122 and the first powder discharge hole 121. Preferably, in the third direction A3, the first powder discharge hole 121 and the vent hole 122 are aligned with each other and spaced apart. Specifically, as shown in FIG. 20 and FIG. 21, the first powder discharge hole 121 and the vent hole 122 are circular holes, the diameter of the first powder discharge hole 121 is equal to the diameter of the vent hole 122, and the first powder discharge hole 121 and the vent hole 122 are completely aligned with each other in the third direction A3. The width of the medicament accommodating portion 62 is equal to the diameter of the first powder discharge hole 121 or the vent hole 122 in the second direction A2. The length of the medicament accommodating portion 62 in the third direction A3 is greater than the width in the second direction A2. Preferably, the length of the medicament accommodating portion 62 exactly completely covers the first powder discharge hole 121 and the vent hole 122 in the third direction A3. In other words, in the third direction A3, the end of the medicament accommodating portion 62 corresponds to the hole wall of the first powder discharge hole 121 away from the vent hole 122, and the other end of the medicament accommodating portion 62 corresponds to the hole wall of the vent hole 122 away from the first powder discharge hole 121. The medicament accommodating portion 62 is completely aligned with the first powder discharge hole 121 and the vent hole 122. In a process of flowing through the vent hole 122 and the first powder discharge hole 121, air may completely flow through the above of the entire medicament accommodating portion 62, to more smoothly and efficiently carry the drug powder in the medicament accommodating portion 62 into the vortex airway 110, thereby ensuring that the drug powder at all positions in the medicament accommodating portion 62 can be discharged out of the medicament accommodating portion 62, and more effectively improving the emptying rate of the drug powder. In this way, the utilization of the drug powder is improved, the problem of incomplete discharge of the drug powder is resolved, and waste of the drug powder is avoided.

In another implementation, the first powder discharge hole 121 and the vent hole 122 may alternatively be arranged in any regular or irregular shape like a rectangular hole, an oval hole, a rhombic hole, or a triangular hole. The shapes of the first powder discharge hole 121 and the vent hole 122 may be the same or may be different. The first powder discharge hole 121 and the vent hole 122 may not be completely aligned with each other. For example, the diameter of the first powder discharge hole 121 is equal to the diameter of the vent hole 122, and only the parts of the first powder discharge hole 121 and the vent hole 122 are aligned with each other in the third direction A3, and the other parts of the first powder discharge hole 121 and the vent hole 122 are staggered with each other. Alternatively, the diameter of the first powder discharge hole 121 is not equal to the diameter of the vent hole 122, so that the first powder discharge hole 121 and the vent hole 122 are partially aligned with each other. In the second direction A2, the width of the medicament accommodating portion 62 may alternatively be greater than or less than the diameter of the first powder discharge hole 121 or the vent hole 122. In the third direction A3, the length of the medicament accommodating portion 62 may alternatively be less than or greater than the size from the end of the first powder discharge hole 121 away from the vent hole 122 to the end of the vent hole 122 away from the first powder discharge hole 121. In other words, the medicament accommodating portion 62 may not be completely aligned with the first powder discharge hole 121 and the vent hole 122, that is, may be partially staggered with the first powder discharge hole 121 and the vent hole 122. The first powder discharge hole 121 and the vent hole 122 may alternatively be spaced apart from each other in another direction, and the first powder discharge hole 121 and the vent hole 122 may alternatively be staggered with each other, provided that the first powder discharge hole 121 and the vent hole 122 each can be in communication with the medicament accommodating portion 62 and airflow can flow through the above of the medicament accommodating portion 62 when flowing from the vent hole 122 to the first powder discharge hole 121. This is not limited in this application. Alternatively, the third air inlet 10 may not be arranged corresponding to a position of a gap between the top wall 114 of the dosage chamber 12 and the bottom wall 113 of the airway chamber 11, and may be arranged at any position of the support 1, provided that the third air inlet 10 can communicate with the external air and the vent hole 122.

Further, in some implementations, because the dose protection mechanism 3 is partially movably arranged in a gap between the dosage chamber 12 and the airway chamber 11 and can move between the third position and the fourth position in the second direction A2, the dose protection mechanism 3 is provided with the first communication hole 311. When the dose protection mechanism 3 is at the third position, the first communication hole 311 is staggered with the first powder discharge hole 121, and the dose protection mechanism 3 blocks the first powder discharge hole 121. In this case, the air inlet channel Q4 is not in communication with the second powder discharge hole 111, to avoid waste of the drug powder caused because the drug powder enters the vortex airway 110 from the first powder discharge hole 121 when the user does not inhale or when the airflow is insufficient during inhalation of the user. When the dose protection mechanism 3 is at the fourth position, the first communication hole 311 is located between the first powder discharge hole 121 and the second powder discharge hole 111, and the first powder discharge hole 121, the first communication hole 311, and the second powder discharge hole 111 are in alignment and communication sequentially to form the second airflow channel Q2. In this case, the dose protection mechanism 3 does not block the air inlet channel Q4, and the third air inlet 10 is in communication with the vent hole 122, so that external air can enter the second powder discharge hole 111 via the third air inlet 10, the vent hole 122, the inside of the medicament accommodating portion 62, the first powder discharge hole 121, and the first communication hole 311 sequentially, so that the air inlet channel Q4 is in communication with the second powder discharge hole 111. The first powder discharge hole 121 and the first communication hole 311 are the part of the second airflow channel Q2 and the part of the air inlet channel Q4. Rotation of the rotating mechanism 2 between the first position and the second position is determined based on the relationship between the negative pressure during inhalation of the user and the preset threshold, to control movement of the dose protection mechanism 3 between the third position and the fourth position in the second direction A2, to implement blocking and communication between the second airflow channel Q2 and the air inlet channel Q4, facilitating improvement of the emptying rate of the drug powder while improving the deaggregation effect on the drug powder.

Further, in some implementations, the dose protection mechanism 3 is further provided with a cutout 34. When the dose protection mechanism 3 is at the third position, the cutout 34 is staggered with the vent hole 122 and/or the third air inlet 10, and the dose protection mechanism 3 blocks the vent hole 122 and/or the third air inlet 10, so that the air inlet channel Q4 is not in communication with the second powder discharge hole 111.

As shown in FIG. 5, FIG. 9A, FIG. 9B, and FIG. 17, in a specific implementation, the cutout 34 is provided on the plate-shaped portion 31 of the dose protection mechanism 3, and the cutout 34 and the first communication hole 311 are aligned with each other and spaced apart in the third direction A3. Specifically, the cutout 34 is a rectangular notch, the first communication hole 311 is a circular hole, the size of the cutout 34 is equal to the aperture of the first communication hole 311 in the second direction A2, and the first communication hole 311 and the cutout 34 are completely aligned with other in the third direction A3. The length of the cutout 34 is greater than the aperture of the vent hole 122 in the third direction A3, and the width of the cutout 34 is equal to the aperture of the vent hole 122 in the second direction A2.

When the airflow is insufficient during inhalation of the user, the negative pressure in the airway chamber 11 is less than or equal to the preset threshold, the rotating mechanism 2 is at the first position, and the dose protection mechanism 3 is at the third position, the first communication hole 311 is completely staggered with the first powder discharge hole 121 and the second powder discharge hole 111, the plate-shaped portion 31 blocks the first powder discharge hole 121, and the second airflow channel Q2 is not in communication. In addition, the cutout 34 is also completely staggered with the vent hole 122 and the third air inlet 10, and the cutout 34 is not in communication with both the vent hole 122 and the third air inlet 10, so that the air inlet channel Q4 is not in communication. In this case, the drug powder in the medicament accommodating portion 62 does not enter the vortex airway 110 via the second powder discharge hole 111, thereby avoiding waste of the drug powder.

When the airflow is sufficient during inhalation of the user, the negative pressure in the airway chamber 11 is greater than the preset threshold, and the dose protection mechanism 3 is at the fourth position, the cutout 34 moves to a position corresponding to the vent hole 122 and is in communication with the vent hole 122. Specifically, in the first direction A1, the cutout 34 and the vent hole 122 are aligned with each other, the projection of the vent hole 122 completely falls with a range of the projection of the cutout 34, and the cutout 34 is in alignment and communication with the vent hole 122. The third air inlet 10 is in alignment and communication with the cutout 34 in the third direction A3, where the third air inlet 10 is located on the side of the cutout 34 away from the first communication hole 311. Preferably, the width of the third air inlet 10 is equal to the width of the cutout 34 in the second direction A2, and the height of the third air inlet 10 is equal to the height of the cutout 34, that is, the thickness of the plate-shaped portion 31 in the first direction A1, so that air entering the air inlet channel Q4 via the third air inlet 10 can completely flow through the cutout 34 and the vent hole 122 and then enter the above of the medicament accommodating portion 62, to carry the drug powder into the second powder discharge hole 111, thereby improving the emptying rate and the air inlet efficiency of the drug powder, and facilitating improvement on the deagglomeration effect on the drug powder.

In another implementation, the cutout 34 may alternatively be arranged in any other shape like a circle, a semi-circle, or a rhombus. The size of the cutout 34 may not be equal to the aperture of the first communication hole 311 in the second direction A2, and the first communication hole 311 and the cutout 34 may be only partially aligned with each other in the third direction A3. Alternatively, the cutout 34 and the first communication hole 311 may be spaced apart from each other in another direction. In the second direction A2, the width of the cutout 34 may alternatively be not equal to the aperture of the vent hole 122, and the width of the third air inlet 10 may alternatively be not equal to the aperture of the vent hole 122. When the dose protection mechanism 3 is at the third position, the first communication hole 311 is in alignment and communication with the second powder discharge hole 111 and the first powder discharge hole 121, and the cutout 34 may be staggered with only the third air inlet 10, and is partially or completely aligned with the vent hole 122, that is, the cutout 34 is in communication with the vent hole 122, and is not in alignment with only the third air inlet 10, so that when the dose protection mechanism 3 is at the third position, the air inlet channel Q4 is not in communication, and external airflow cannot flow through the air inlet channel Q4 and then enter the second powder discharge hole 111 after passing through the third air inlet 10. Alternatively, when the dose protection mechanism 3 is at the third position, the cutout 34 may be staggered with only the vent hole 122, and is partially or completely aligned with the third air inlet 10, so that the air inlet channel Q4 is not in communication. When the dose protection mechanism 3 is at the fourth position, the cutout 34 may be partially aligned with the third air inlet 10 and/or the vent hole 122, so that the air inlet channel Q4 is in communication with the second powder discharge hole 111.

In another implementation, the dose protection mechanism 3 of the airway structure may alternatively be provided with only the first communication hole 311 instead of the cutout 34, or may be provided with only the cutout 34 instead of the first communication hole 311. Communication and blocking between the air inlet channel Q4 and the second powder discharge hole 111 are controlled based on movement of the dose protection mechanism 3 between the third position and the fourth position, thereby reducing waste of the drug powder while improving the emptying rate of the drug powder.

In some embodiments, further referring to FIG. 2, FIG. 4A, and FIG. 4B, the dry powder inhaler 800 further includes a supply reel 400, a cover take-up reel 500, a dosage wheel 600, and a base take-up reel 700. The supply reel 400, the cover take-up reel 500, the dosage wheel 600, and the base take-up reel 700 are all mounted on the support 1.

The supply reel 400 is configured to supply the drug tape 6. The supply reel 400 includes a drug tape mounting shaft 401, and the drug tape mounting shaft 401 is used for mounting the drug tape 6. In the length direction of the drug tape 6, at the end of the drug tape 6, the base tape 61 and the cover tape are in a peeled state. The cover take-up reel 500 is configured to peel the cover tape from the base tape 61 to expose the medicament accommodating portion 62 on the base tape 61, and is configured to take up the cover tape from which the base tape 61 is peeled. The dosage wheel 600 is arranged in the dosage chamber 12. The dosage wheel 600 is configured to deliver the base tape 61 obtained after the cover tape is peeled by using the cover take-up reel 500, to deliver the medicament accommodating portion 62 to the position corresponding to the first powder discharge hole 121, so that the drug powder in the medicament accommodating portion 62 can be discharged via the first powder discharge hole 121 during inhalation of the user. The base take-up reel 700 is configured to take up the base tape 61 obtained after the cover tape is peeled by using the cover take-up reel 500 and the drug powder in the medicament accommodating portion 62 is inhaled by the user.

The support 1 is further provided with a drug tape accommodating groove 7, at least the supply reel 400 is located in the drug tape accommodating groove 7, and the drug tape accommodating groove 7 is for accommodating at least the drug tape 6 that is mounted on the drug tape mounting shaft 401 and that is taken up. As shown in FIG. 2, FIG. 12, and FIG. 13, in an implementation, the supply reel 400 and the base take-up reel 700 are located in the drug tape accommodating groove 7, and are spaced apart from each other and do not interfere with each other. In another implementation, if the drug tape accommodating groove 7 has sufficiently large space, the supply reel 400, the base take-up reel 700, and the cover take-up reel 500 may all be arranged in the drug tape accommodating groove 7, and may be designed based on requirements.

The drug tape 6 includes the base tape 61 and the cover tape (not shown in the figure). The base tape 61 is provided with the medicament accommodating portion 62 (namely, a drug reservoir) for accommodating a powdered drug. The cover tape is adhered to the surface of the base tape 61 and covers the medicament accommodating portion 62. During use, the cover tape is peeled from the base tape 61. The dosage wheel 600 is accommodated in the dosage chamber 12. The outer side surface of the dosage wheel 600 is provided with a plurality of dosage grooves (not marked in the figure). The base tape 61 from which the cover tape is peeled is wrapped around the dosage wheel 600, so that the medicament accommodating portion 62 is located in the dosage groove and the top of the medicament accommodating portion 62 is exposed. In this way, the medicament accommodating portion 62 is used as the part of the air inlet channel Q4, facilitating inhalation of the user to the drug powder in the medicament accommodating portion 62.

A gear may be arranged in each of the supply reel 400, the cover take-up reel 500, the dosage wheel 600, and the base take-up reel 700, and transmission may be performed by using a plurality of gears to form a gear set, thereby implementing respective functions. In an implementation, the rotation center of the cover body 300 may be linked with a gear set. Rotation of the cover body 300 between the seventh position and the eighth position drives the gear set to move, so that the supply reel 400, the cover take-up reel 500, the dosage wheel 600, and the base take-up reel 700 implement the functions. In addition, the limit on the translation mechanism 5 of the breath triggering device 100 may also be released, facilitating a subsequent breath triggering process. In other words, in a process in which the cover body 300 rotates from the seventh position to the eighth position (that is, a cover opening process), linking between a plurality of submodules is implemented. A linking action of related modules may be implemented by using a cover opening operation, thereby simplifying a user operation, and improving user compliance.

Specifically, the dry powder inhaler 800 may include a ratchet (not shown in the figure) and a ratchet gear (not shown in the figure). The ratchet and the ratchet gear each are provided with a plurality of pawls and are assembled and connected with each other. The ratchet gear is in meshing engagement with the foregoing gear set. The rotation center of the cover body 300 is connected to the center of the ratchet. When the cover body 300 rotates from the seventh position to the eighth position, because the ratchet and the ratchet gear are in meshing engagement with each other by using the pawls, the cover body 300 drives the ratchet and the ratchet gear to synchronously rotate, and then the ratchet gear drives the gear set to rotate, so that the plurality of submodules implement the functions. In a process in which the cover body 300 rotates from the eighth position to the seventh position, due to the pawls arranged on the ratchet and the ratchet gear, the cover body 300 drives only the ratchet connected to the cover body 300 to rotate in the reverse direction, and the ratchet gear does not rotate, so that the gear set of the supply reel 400, the cover take-up reel 500, the dosage wheel 600, and the base take-up reel 700 also does not rotate, thereby avoiding loosening or inadequate wrapping of the drug tape, the base tape, or the cover tape.

In another implementation, an independent drive member may be arranged to drive the gear set to move, so that the components implements functions. Gears in the supply reel 400, the cover take-up reel 500, the dosage wheel 600, and the base take-up reel 700 may synchronously rotate or asynchronously rotate, and may be designed as required.

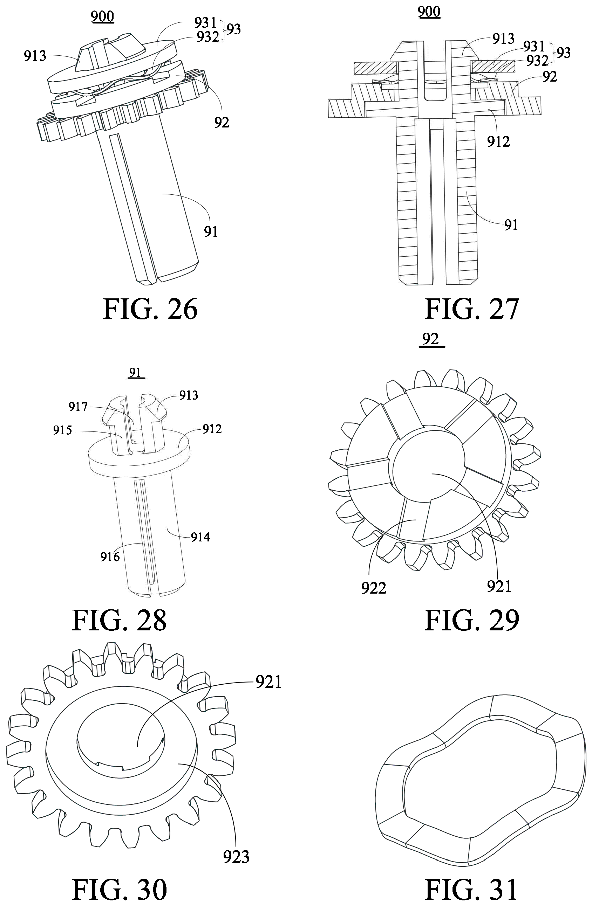

Referring to FIG. 26 to FIG. 31, FIG. 26 is a schematic structural diagram of an embodiment of a take-up device according to this application; FIG. 27 is a cross sectional schematic view of the take-up device provided in FIG. 26; FIG. 28 is a schematic structural diagram of a tape take-up rod of the take-up device provided in FIG. 26; FIG. 29 is a bottom schematic structural diagram of a tape take-up gear of the take-up device provided in FIG. 26; FIG. 30 is a bottom schematic structural diagram of the tape take-up gear provided in FIG. 29; and FIG. 31 is a schematic structural diagram of a wave washer of the take-up device provided in FIG. 25.

Referring to FIG. 26 and FIG. 27, this application further provides a take-up device 900. At least one of the cover take-up reel 500 and the base take-up reel 700 of the dry powder inhaler 800 may be the foregoing take-up device 900. The take-up device 900 includes a tape take-up rod 91, a tape take-up gear 92, and a washer 93. The tape take-up rod 91 includes a rod-shaped portion 911, a first clamping portion 912, and a second clamping portion 913. The first clamping portion 912 and the second clamping portion 913 are spaced apart from each other on the outer side surface of the rod-shaped portion 911. The rod-shaped portion 911 is used for taking up a web, where the web may be a base tape 61 or a cover tape. The tape take-up gear 92 is provided with a central hole 921. The tape take-up gear 92 is sleeved on the outer side surface of the rod-shaped portion 911 and is located between the first clamping portion 912 and the second clamping portion 913. In other words, the first clamping portion 912 and the second clamping portion 913 limit the tape take-up gear 92. The hole wall of the central hole 921 is adhered to the outer side surface of the rod-shaped portion 911, and the surface of the tape take-up gear 92 close to the first clamping portion 912 is arranged in contact with the first clamping portion 912. The washer 93 is mounted between the tape take-up gear 92 and the second clamping portion 913, and is separately arranged in contact with the tape take-up gear 92 and the second clamping portion 913.

It may be understood that, the first clamping portion 912 and the second clamping portion 913 limit the mounting of the tape take-up gear 92 on the tape take-up rod 91, the tape take-up gear 92 and the tape take-up rod 91 have simpler structures and smaller volumes, and the diameter of the tape take-up rod 91 is smaller than the diameter of the tape take-up gear 92. In this way, more webs can be taken up, thereby effectively saving space and simplifying the structure of the take-up device 900. In this way, the take-up device 900 is applicable to a small space structure, and has a wider application range. In addition, the hole wall of the central hole 921 of the tape take-up gear 92 is adhered to the outer side surface of the rod-shaped portion 911, and the surface of the tape take-up gear 92 close to the first clamping portion 912 is arranged in contact with the first clamping portion 912, so that a contact area between the tape take-up gear 92 and the tape take-up rod 91 is larger, and the friction force is stronger, which is more beneficial for the tape take-up gear 92 to drive the tape take-up gear 91 to rotate to take up the web. In addition, when the tape take-up rod 91 is excessively loaded, the tape take-up gear 92 may slide relative to the tape take-up rod 91, where the tape take-up gear 92 still rotates, and the tape take-up rod 91 may stop rotating or may rotate at the speed lower than the speed of the tape take-up gear 92, so that the tape take-up gear 92 may slide relative to the tape take-up rod 91. Therefore, the load of the tape take-up rod 91 is effectively reduced by using the sliding friction force between the tape take-up gear 92 and the tape take-up rod 91, to achieve a take-up compensation effect, to avoid the following problem: for example, because the tape take-up rod 91 is still taking up the web when the load of the tape take-up rod 91 is excessively large, the tape take-up rod 91 takes up the web excessively tightly, and then the web is broken. After take-up compensation is performed on the tape take-up rod 91, the tape take-up rod 91 can be still driven by the tape take-up gear 92 to synchronously rotate with the tape take-up gear 92, to continue to take up the web.

As shown in FIG. 26 and FIG. 27, in an implementation, the washer 93 includes a rigid washer 931 and an elastic washer 932. The elastic washer 932 is located between the tape take-up gear 92 and the rigid washer 931. The elastic washer 932 is separately arranged in contact with the tape take-up gear 92 and the rigid washer 931. In a specific implementation, the rigid washer 931 is a flat washer, and the elastic washer 932 is a wave washer. It may be understood that, both the rigid washer 931 and the elastic washer 932 are arranged. During assembly, the tape take-up gear 92 may be compressed by using the elastic washer 932, so that contact between the tape take-up gear 92 and the tape take-up rod 91 is closer, and the tape take-up rod 91 is driven by rotation of the tape take-up gear 92 to synchronously rotate, to take up the web. Arranging the rigid washer 931 may also cause the take-up device 900 to have higher assembly stability. Arranging the elastic washer 932 in contact with the rigid washer 931 may also reduce an energy loss of the entire take-up device 900 during movement of the tape take-up gear 92.

Specifically, the side of the wave washer facing the tape take-up gear 92 is provided with a peak. As shown in FIG. 29, the surface of the tape take-up gear 92 close to the wave washer is provided with a limiting groove 922 corresponding to the peak, and the peak is correspondingly arranged in the limiting groove 922, so that the tape take-up gear 92 and the wave washer synchronously rotate, to avoid the problem: when the tape take-up gear 92 and the wave washer slide relative to each other, the sliding friction force between the tape take-up gear 92 and the wave washer causes an energy loss of the take-up device 900.

Referring to FIG. 26, FIG. 27, and FIG. 30, the surface of the tape take-up gear 92 away from the washer 93 is provided with a receiving groove 923, the central hole 921 is arranged on the bottom wall 113 of the receiving groove 923, and the first clamping portion 912 is inserted in the receiving groove 923. Specifically, the surface of the first clamping portion 912 close the second clamping portion 913 is adhered to the bottom surface of the receiving groove 923, and/or the side surface of the first clamping portion 912 is adhered to the side surface of the receiving groove 923. Preferably, the surface of the first clamping portion 912 close the second clamping portion 913 is adhered to the bottom surface of the receiving groove 923, and the side surface of the first clamping portion 912 is adhered to the side surface of the receiving groove 923. It may be understood that in the foregoing implementation, the contact area between the tape take-up gear 92 and the tape take-up rod 91 is larger, and the static friction force is stronger, which is more beneficial for the tape take-up gear 92 to drive the tape take-up rod 91 to synchronously rotate. In addition, when the load of the tape take-up rod 91 is excessively large, the load of the tape take-up rod 91 may be reduced more rapidly through staggered movement between the tape take-up rod 91 and the tape take-up gear 92, thereby more efficiently achieving the take-up compensation effect, and avoiding the problem that the web is damaged because the web is taken up excessively tightly.

Referring to FIG. 26, FIG. 27, and FIG. 28, the rod-shaped portion 911 of the tape take-up rod 91 includes a first rod segment 914 and a second rod segment 915 connected to each other. The first clamping portion 912 is located at a connection between the first rod segment 914 and the second rod segment 915. The second clamping portion 913 is arranged at the end of the second rod segment 915 away from the first clamping portion 912. The first rod segment 914 is used for taking up the web. The tape take-up gear 92 and the washer 93 are sleeved on the second rod segment 915.

In a specific implementation, the rod-shaped portion 911 is in a cylindrical shape, the first clamping portion 912 is in an annular shape, the central hole 921 is a circular hole, the receiving groove 923 is a circular groove, the surface of the second clamping portion 913 away from the first clamping portion 912 is an inclined surface, and the inclined surface is arranged obliquely to an axis of the rod-shaped portion 911. In addition, the diameter of the second clamping portion 913 gradually increases from the end of the second clamping portion 913 away from the first clamping portion 912 to the end of the second clamping portion 913 close to the first clamping portion 912, and the diameter of the end of the second clamping portion 913 close to the first clamping portion 912 is smaller than the diameter of the first clamping portion 912. It may be understood that, the surface of the second clamping portion 913 away from the first clamping portion 912 is arranged as the inclined surface, so that it may be more convenient to assemble the washer 93 and the tape take-up gear 92 to the tape take-up rod 91, thereby improving assembly efficiency. In addition, the diameter of the end of the second clamping portion 913 close to the first clamping portion 912 is smaller than the diameter of the first clamping portion 912, to avoid that the tape take-up gear 92 cannot be limited between the first clamping portion 912 and the second clamping portion 913 because the central hole 921 of the tape take-up gear 92 passes through the first clamping portion 912.

The first rod segment 914 is in a hollow cylindrical shape. The side wall of the first rod segment 914 is provided with at least one first slot 916, and the first slot 916 extends in the axial direction of the rod-shaped portion 911. As shown in FIG. 28, in a specific implementation, the side wall of the first rod segment 914 is provided with two first slots 916, and the first slots 916 are used for allowing the end portion of the web to be threaded, so that the first rod segment 914 takes up the web. In addition/alternatively, the second rod segment 915 is in a hollow cylindrical shape, and the side wall of the second rod segment 915 is provided with at least one second slot 917. As shown in FIG. 28, in a specific implementation, the side wall of the second rod segment 915 is provided with two second slots 917, and the second slots 917 extend from the end of the second rod segment 915 away from the first rod segment 914 to the first clamping portion 912. During assembly of the take-up device 900, the second slot 917 may facilitate inward contraction of the second rod segment 915, so that the tape take-up gear 92 and the washer 93 can be more smoothly assembled from the end of the second clamping portion 913 to between the second clamping portion 913 and the first clamping portion 912, thereby improving assembly efficiency. In addition, the second rod segment 915 returns to the original shape after assembly is completed, facilitating limiting on the tape take-up gear 92 and the washer 93.

The foregoing descriptions are merely embodiments of this application but are not intended to limit the patent scope of this application. Any equivalent modifications made to the structures or processes based on the content of this specification and the accompanying drawings of this application for direct or indirect use in other related technical fields shall also fall within the patent protection scope of this application.

## Claims

1. A rotating mechanism, used in a breath triggering device, wherein the rotating mechanism comprises:
a rotating shaft, wherein the outer side surface of the rotating shaft is provided with a notch; and
a baffle, connected to the outer side surface of the rotating shaft, wherein
the rotating mechanism is rotatable between a first position and a second position, and blocks a first airflow channel of the breath triggering device when the rotating mechanism is at the first position; and when the rotating mechanism moves from the first position to the second position, the baffle rotates around the rotating shaft to open the first airflow channel.

2. The rotating mechanism of claim 1, wherein a protruding rod is connected to the end of the rotating shaft; and
the side surface of the end of the rotating shaft is provided with a groove, the end of the protruding rod is connected to the side wall of the groove, and an included angle is provided between the protruding rod and the baffle.

3. The rotating mechanism of claim 1, wherein the baffle has the first surface, the first surface is provided with a flange, and the flange is arranged at the end of the baffle away from the rotating shaft, and extends from one side to the other side along the edge of the end of the baffle away from the rotating shaft.

4. The rotating mechanism of claim 3, wherein a chamfer is provided on the end of the flange away from the rotating shaft.

5. The rotating mechanism of claim 3, wherein the baffle further has the second surface opposite to the first surface, and the second surface is provided with a stop member; the stop member is arranged at the end of the baffle away from the rotating shaft, and extends from one side to the other side along the edge of the end of the baffle away from the rotating shaft; and in the direction parallel to the second surface, the end of the stop member is connected to the second surface, and the other end of the stop member extends out of the second surface to form a limiting portion.

6. A dose protection mechanism, used in a breath triggering device, wherein
the dose protection mechanism is movable between a third position and a fourth position; the dose protection mechanism comprises a plate-shaped portion, and the plate-shaped portion is provided with a first communication hole and a second communication hole spaced apart from each other;
when the dose protection mechanism is at the third position, the plate-shaped portion blocks a second airflow channel of the breath triggering device, and the second communication hole is in communication with external air and an airway chamber of the breath triggering device; and when the dose protection mechanism is at the fourth position, the first communication hole is in communication with the second airflow channel, and the plate-shaped portion is used for blocking the airway chamber.

7. The dose protection mechanism of claim 6, wherein the dose protection mechanism further comprises a body portion, and a limiting member is provided on the body portion.

8. The dose protection mechanism of claim 7, wherein the limiting member is a protruding post obliquely arranged on the surface of the body portion, and the protruding post has the free end.

9. The dose protection mechanism of claim 7, wherein the first communication hole and the second communication hole are circular holes, and are aligned with each other and spaced apart in the movement direction of the dose protection mechanism; and
the body portion is further provided with an accommodation groove.

10. A breath triggering device, comprising:
an airway chamber;
a rotating mechanism, rotatable between a first position and a second position; and
a dose protection mechanism, movable between a third position and a fourth position,
wherein
when a negative pressure in the airway chamber of the breath triggering device is greater than a preset threshold, external air pressure pushes the rotating mechanism to rotate from the first position to the second position, the rotating mechanism releases a limit on the dose protection mechanism, and the dose protection mechanism moves from the third position to the fourth position; and/or
when the negative pressure in the airway chamber of the breath triggering device is greater than the preset threshold, the rotating mechanism and the dose protection mechanism move, for a communication manner of an airflow channel of the breath triggering device to be changed.

11. The breath triggering device of claim 1, wherein
the rotating mechanism is the rotating mechanism of any one of claims 1 to 5;
the dose protection mechanism is the dose protection mechanism of any one of claims 6 to 9; and
the breath triggering device further comprises a dosage chamber spaced apart from the airway chamber, wherein the airway chamber is in communication with the dosage chamber via the second airflow channel, and the airway chamber is in communication with the external air via the first airflow channel or a third airflow channel, wherein
when a negative pressure in the airway chamber is greater than the preset threshold, the external air pressure pushes the rotating mechanism to rotate from the first position to the second position, for the first airflow channel to be opened; when the dose protection mechanism is at the third position, the second airflow channel is blocked, the airway chamber is not in communication with the dosage chamber, the third airflow channel is opened, and the airway chamber is in communication with the external air via the third airflow channel; and when the dose protection mechanism is at the fourth position, the second airflow channel is opened, to communicate the airway chamber with the dosage chamber, and the third airflow channel is blocked.

12. The breath triggering device of claim 11, further comprising:
a support, provided with the airway chamber and the dosage chamber spaced apart from each other, wherein the rotating mechanism is rotatably arranged on the support, and the dose protection mechanism is movably arranged on the support; and
an elastic member, arranged in contact with the dose protection mechanism, and configured to drive the dose protection mechanism to move from the third position to the fourth position, wherein
when the rotating mechanism rotates from the first position to the second position, the rotating mechanism releases the limit on the dose protection mechanism, for the elastic member to drive the dose protection mechanism to move from the third position to the fourth position.

13. The breath triggering device of claim 12, wherein
the dosage chamber is provided with a first powder discharge hole, and the airway chamber is provided with a second powder discharge hole; the first powder discharge hole and the second powder discharge hole are aligned with each other and spaced apart, to form the part of the second airflow channel; and the support is further provided with a third communication hole, and the third communication hole and the airway chamber are spaced apart from each other, wherein
when the dose protection mechanism is at the third position, the first communication hole is staggered with the first powder discharge hole, and the first powder discharge hole is blocked; when the dose protection mechanism is at the third position, the second communication hole is in alignment and communication with the third communication hole to form the part of the third airflow channel; and when the dose protection mechanism is at the fourth position, the first communication hole is located between the first powder discharge hole and the second powder discharge hole, for the first powder discharge hole, the first communication hole, and the second powder discharge hole to be in alignment and communication sequentially to form the second airflow channel.

14. The breath triggering device of claim 12, further comprising a translation mechanism, wherein the translation mechanism is movably arranged on the support and is movable between a fifth position and a sixth position; and the translation mechanism is separately arranged in contact with the rotating mechanism and the dose protection mechanism, wherein
the translation mechanism moves from the sixth position to the fifth position, to drive the dose protection mechanism to be reset from the fourth position to the third position, and drive the rotating mechanism to be reset from the second position to the first position.

15. The breath triggering device of claim 14, wherein the dose protection mechanism is slidably arranged on the support; the dose protection mechanism comprises the body portion, and the plate-shaped portion is connected to the side of the body portion away from the elastic member; the end of the rotating shaft is connected to the protruding rod, and the body portion is further provided with the accommodation groove;
the translation mechanism comprises a support member and a push rod connected to each other, the support member abuts against the protruding rod, and the push rod is at least partially arranged in the accommodation groove; and
when the translation mechanism moves from the sixth position to the fifth position, the support member pushes the protruding rod, to reset the rotating mechanism from the second position to the first position, and the push rod pushes the body portion, to reset the dose protection mechanism from the fourth position to the third position.

16. The breath triggering device of claim 11, wherein the airway chamber and the dosage chamber are spaced apart from each other in the first direction; the first airflow channel and the rotating mechanism are arranged on the side of the airway chamber in the second direction; the dose protection mechanism slides in the second direction; the shaft direction of the rotating shaft is the third direction; and
the first direction, the second direction, and the third direction are perpendicular to each other.

17. A dry powder inhaler, comprising:
a housing assembly, comprising a housing and a mouthpiece; and
a breath triggering device, arranged in the housing, wherein the breath triggering device is the breath triggering device of any one of claims 10 to 16, wherein
the mouthpiece is in communication with the airway chamber.

18. The dry powder inhaler of claim 17, wherein the breath triggering device is the breath triggering device of claim 14 or 15; and the dry powder inhaler further comprises:
a cover body, rotatably connected to the housing assembly, wherein the cover body is rotatable between a seventh position and an eighth position, and drives the translation mechanism to move from the sixth position to the fifth position when the cover body rotates from the eighth position to the seventh position; when the cover body is at the seventh position, the cover body covers the mouthpiece and limits the rotating mechanism to the first position via the translation mechanism; and when the cover body is at the eighth position, the cover body exposes the mouthpiece and releases the limit on the translation mechanism.

19. The dry powder inhaler of claim 18, wherein
the cover body is connected to the translation mechanism, and drives the translation mechanism to slide between the fifth position and the sixth position based on that the cover body rotates between the seventh position and the eighth position; or
the support is further provided with an elastic arm, wherein the elastic arm abuts against the translation mechanism when the translation mechanism is at the fifth position; and the elastic arm drives the translation mechanism to slide from the fifth position to the sixth position when the cover body is at the eighth position; or
the translation mechanism is driven to slide from the fifth position to the sixth position when the rotating mechanism rotates from the first position to the second position; or
the translation mechanism is driven to slide from the fifth position to the sixth position when the dose protection mechanism moves from the third position to the fourth position.
